(19) Europäisches Patentamt
European Patent Office
Office européen des brevets

(11) EP 3 039 160 B1

(12) EUROPEAN PATENT SPECIFICATION

(45) Date of publication and mention of the grant of the patent:
20.09.2017 Bulletin 2017/38

(21) Application number: 14781929.6

(22) Date of filing: 27.08.2014

(51) Int Cl.:
$C12Q$ 1/68 (2006.01)

(86) International application number:
PCT/GB2014/052595

(87) International publication number:
WO 2015/028792 (05.03.2015 Gazette 2015/09)

(54) OLIGONUCLEOTIDES COMPRISING A SECONDARY STRUCTURE AND USES THEREOF

OLIGONUKLEOTIDE MIT EINER SEKUNDÄREN STRUKTUR UND VERWENDUNGEN DAVON

OLIGONUCLÉOTIDES COMPRENANT UNE STRUCTURE SECONDAIRE ET LEURS UTILISATIONS

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 27.08.2013 GB 201315234

(43) Date of publication of application:
06.07.2016 Bulletin 2016/27

(73) Proprietor: LGC Limited
Teddington
Middlesex TW11 0LY (GB)

(72) Inventors:
• FRENCH, David
Teddington
Middlesex TW11 0LY (GB)
• DEBENHAM, Paul
Teddington
Middlesex TW11 0LY (GB)

(74) Representative: Potter Clarkson LLP
The Belgrave Centre
Talbot Street
Nottingham NG1 5GG (GB)

(56) References cited:
WO-A1-2005/030987     WO-A2-2005/007815
WO-A2-2006/020933     GB-A- 2 456 670
US-A- 6 114 121

**Description**

The present invention relates to oligonucleotides, and in particular their uses as probes in the detection of hybridisation events and nucleic acid detection and/or discrimination using detectable labels (such as visually detectable labels).

Probe technologies based on oligonucleotides labelled with detectable labels (such as fluorophores) are long-established, especially with the fluorophore as an addition at the 5' phosphate terminal. Such terminal labelling is inexpensive and hence the development and application of such probes is wide-spread across molecular biology applications. In contrast, an advantageous design of probes in which multiple fluorophores can be attached to the nucleoside bases or sugars within the oligonucleotide sequence (i.e. internally labelled), such as HyBeacon probes (French *et al.* (2001), WO 01/73118, WO 07/010268), have been less widely applied because of the inherently more expensive nature of such internal fluorophore additions. In terminally labelled oligonucleotides, there is typically the need for a separate quencher molecule to also be present in order to prevent a signal before the oligonucleotide hybridises to a target polynucleotide. Internally labelled oligonucleotides (such as those described in WO 01/73118, WO 07/010268) do not typically require the presence of a separate quencher because the single stranded sequence of the oligonucleotide probe serves to act as a quencher for the label (DNA quenching).

The long established field of using labelled nucleic acid oligonucleotides as a reporter for the presence of a target nucleic acid sequence (DNA or RNA) utilises the direct nucleotide sequence complementarity of an oligonucleotide based probe with its target nucleic acid sequence and its unique match achieved by sufficient shared sequence compared to the genomic background.

Oligonucleotide hybridisation to nucleic acid sequences removes the DNA quenching and dye-dye interactions and causes increased fluorescence emission (WO 01/73118, WO 07/010268). Changes in fluorescence emission allow detection of specific nucleic acid sequences and the stability of the interactions between probes and targets permit polymorphic sequences to be discriminated on the basis of length and sequence (French *et al.* 2001, French *et al.* 2002, French *et al.* 2007, French *et al.* 2008).

The deliberate use of either partial sequence complementarity or the use of additional oligonucleotides providing intermediary hybridisation events between the probe and its target is unnecessary unless achieving a particular additional molecular end point alongside the probe-target interaction is desired.

In the example of a fluorescent oligonucleotide probe, the important incremental benefit of the direct sequence complementarity found in conventional probe-target hybridised pairing is that any sequence variation in the target, such as a single nucleotide polymorphism (SNP), is directly disruptive to that hybridisation and hence the fluorescent output of the probe.

DNA stem-loop sequences in oligonucleotide probes are already known to be useful in signal generation and target detection, predominantly such that the stem-loop structure is lost when the oligonucleotide hybridizes to a target polynucleotide (i.e. the oligonucleotide no longer possesses a secondary structure).

"Molecular beacons" are single-stranded oligonucleotides that form stem-loop structures (Tyagi & Kramer, 1996; Tyagi et al. 1998). The stem comprises two complementary nucleic acid sequences that are positioned on either side of a probing sequence. One of the stem sequences is typically labelled with a fluorophore and the other is typically labelled with a non-fluorescent quencher. The stem-loop structure brings the fluorophore and quencher moieties into close proximity to efficiently quench fluorescence emission. The loop components of molecular beacon oligonucleotides are complementary to target nucleic acids and hybridisation causes dissociation of the stem-loop, since intermolecular probe/target hybridisation is more stable than the intramolecular interaction between oligonucleotide stem sequences. Separation of fluorophore and quencher labels upon probe hybridisation due to disassociation of the stem-loop structure causes increased signal emission. Molecular beacon oligonucleotides can be used for real-time PCR and melting curve analysis.

Similar to molecular beacons, Knemeyer et al. (2000) describe oligonucleotides (Smart probes) that have stem-loop structures but achieve fluorescence quenching by photoinduced intramolecular electron transfer to guanine residues in one of the oligonucleotide stem sequences. A fluorophore (such as oxazine dye JA242) attached to the end of one oligonucleotide stem sequence is brought into close proximity with G-rich sequence in the complementary stem sequence of the hairpin. Probe hybridisation to target sequences separates the fluorophore from the G-rich sequence causing fluorescence enhancement.

Scorpion™ primers comprise a probing sequence that is located within a stem-loop structure, with complementary stem sequences positioned on either side of the probing sequence (Whitcombe et al. 1999; Thelwell et al. 2000). A fluorophore moiety attached to the 5' end of the oligonucleotide is quenched by an internal quencher modification attached at the 3' end of the stem-loop. The stem-loop sequence is attached to the 5' end of a PCR primer via a PCR stopper such as hexaethylene glycol (HEG). Extension of the primer component generates the target sequence for the probing component of the oligonucleotide. Intramolecular probe hybridisation to amplified target causes dissociation of the stem-loop structure and increased fluorescence emission.

Molecular beacons, Smart probes and Scorpion™ primers use hairpin structures with stem sequences located on both ends of a probing sequence. The loops bind to the target polynucleotide but the stem sequences do not interact with the target.

Satterfield et al. (2007) and US2009/0305264 describe oligonucleotides (Tentacle probes™) that have stem-loop sequences similar to molecular beacons (with fluorophore and quencher moieties brought into close proximity in a hairpin) attached to a capture probe sequence via a linker. The capture probe component binds to target sequence and brings the stem-loop component into close proximity with downstream target. The probe sequence of the hairpin then binds to target sequence to separate fluorophore and quencher labels and increase fluorescence emission. The addition of the capture probe sequence is reported to improve hybridisation kinetics.

WO 2010/101947 also describes stem-loop oligonucleotides used to detect nucleic acid sequence (also a form of tentacle probe™). Hairpin structures are formed at 5' or 3' ends to bring two labels into close proximity in the absence of target (for dye quenching or FRET). One of the oligonucleotide ends (5' or 3') is complementary to an internal region of the probe sequence and the loop of the hairpin is complementary to a nucleic acid target. The other oligonucleotide end (3' or 5') extends beyond the hairpin to initiate target hybridisation and aid hybridisation of the probing sequence within the stem-loop structure. The additional probing sequence outside of the hairpin improves hybridisation kinetics. Target hybridisation causes oligonucleotide labels to be separated for removal of fluorescence quenching or FRET interactions.

Nazarenko et al. (1997) describe oligonucleotides (Sunrise primers or Amplifluor™) that have a stem-loop structure attached to the 5' end of a primer. A fluorophore is attached to the 5' end of the oligonucleotide and a quencher is positioned close to the 3' end of the primer. Formation of the stem-loop structure brings fluorophore and quencher moieties into close proximity on the stem causing efficient fluorescence quenching. PCR amplification causes Sunrise primers to be incorporated into amplification products, preventing formation of the stem-loop and causing separation of fluorophore and quencher moieties. Fluorescence emission is increased in the presence of amplified target sequences. However, non-specific amplification arising from primer dimers can also result in enhanced fluorescence emission.

Nazarenko et al. (2002) describe oligonucleotides (LUX primers™) that are labelled with a single fluorophore positioned close to the 3' end of a primer. A sequence of 5-7 nucleotides is positioned at the 5' end of the oligonucleotide which is complementary to the 3' end of the primer to create a hairpin. Oligonucleotides do not comprise a quencher modification but use DNA quenching in the hairpin to reduce signal emission in the absence of target. PCR amplification causes oligonucleotides to be incorporated into amplification products, preventing formation of the stem-loop structure and removing the DNA quenching imposed by the hairpin.

Tentacle probes™, sunrise primers and LUX primere™ all use stem-loop sequences for target detection by using hairpin structures to bring two labels (or a label and quenching nucleotide sequence) into close proximity in the absence of target. Probe hybridisation to target nucleic acids causes oligonucleotide labels to be separated allowing measurement of altered fluorescence emission. In each, the target specific nucleotide sequence is incorporated into the hairpin structure.

US 2007/0015176 teaches detector probes with varying secondary structures, each including at least one stem-loop structure. The detector probes of US 2007/0015176 all require there to be a double stranded nucleotide structure comprising of two separate oligonucleotides.

US 2006/0216692 describes nucleic acid constructs labelled with both a fluorescent label and a quencher molecule. The labelled oligonucleotides include at least one stem-loop structure and function as switchable constructs that switch between two alternative secondary structures. The first secondary structure comprises two stem-loop structures and brings the quencher and fluorophore into close proximity with each other, the alternative secondary structure, which is formed on hybridising a target conformation is an extended stem-loop structure (with an elongated stem) such that the fluorophore and quencher are no longer in close proximity and a signal is detectable. The portion of the oligonucleotide that hybridises to a target is located in the loop portion of one of the stem-loop structures (in the double stem-loop conformation) and in the stem of the extended single stem-loop structure.

There have also been studies looking at preventing primer oligonucleotides from participating in DNA polymerase primer extension until suitable single-stranded DNA extension temperatures are provided within a reaction mix. One approach is to contain the 3' terminal of the primer in a short double-stranded configuration with a sequence at the 5' end of the primer which will melt apart for priming at a suitable temperature. The configuration of the primer may be as a loop (Ailenberg and Silverman (2000), or as a hairpin (Kaboev et al. 2000; Nazarenko et al. 2002). These structures are intended to enable 3' extension at the preferred polymerase extension temperature. Nazarenko et al. (2002) observe that primer-dimer artifacts may affect probe-based methods because the primer-dimer formation suppresses formation of the specific amplicon.

US 2006/0088856 describes oligonucleotides that are labelled with a fluorophore and a quencher, with either the fluorophore or quencher attached to the 3' nucleotide. The oligonucleotides of US 2006/0088856 exhibit substantially constant levels of signal emission when both single-stranded and bound to target nucleic acids. Oligonucleotides hybridise during target amplification and are cleaved by the 5'-3' forward processitivity functionality of standard DNA polymerases to separate fluorophore and quencher labels. Cleavage to separate the fluorophore and quencher is the action which generates a detectable signal. US 2006/0088856 exemplify oligonucleotides with a 5' hairpin that improve real-time amplification and detection of targets (reducing detection Cts by 2-3 cycles) when compared with standard linear 5'-3' hydrolysis probes. The hairpin structures described are intended to improve the speed and efficiency of real-time amplification which can consequently increase test sensitivity with rapid PCR thermal cycling.

WO 2006/020933 describes a hairpin containing probe utilising a quencher-fluorophore pair which can only be detected after cleavage of the probe. WO 2005/007818 describes a labelled probe containing a hairpin wherein the probe is extended at the 3' end in PCR. WO 99/24621, EP 0 881 302 and WO 98/02449 all describe probes containing a labelled hairpin, with EP 0 881 302 and WO 98/02449 also disclose utilising a quencher-fluorophore pair which can only be detected after their separation. In addition, WO 98/02449 requires the probe to be extended at the 3' end in PCR.

Oligonucleotide probes are typically included within PCR or isothermal amplification reactions so as to detect the amplified target sequences whilst not directly being engaged in the amplification process. In this respect, it is standard practice to add a protective cap by modification of the 3' end of the oligonucleotide to reduce polymerase extension from probes. However polymerases with strong 3'-5' exonuclease activity can cause these 3' modifications to be removed such that the oligonucleotide probes can be extended and act as primers. This exonuclease activity and/or the extension of the oligonucleotide probe can reduce the effectiveness of the visually detectable label(s).

There is herein described a novel variation in the design of labelled oligonucleotide probes in order to improve oligonucleotide probe function by preventing 3'-5' exonuclease cleavage and polymerase mediated extension of oligonucleotide probes.

In a first aspect of the invention there is provided a single stranded oligonucleotide having a 5' end and a 3' end, said oligonucleotide comprising a first and second section, the first section being positioned 5' of the second section; and wherein:

(i) the first section is labelled internally with at least two detectable labels, wherein the at least two detectable labels are the same, and is capable of hybridising to a target polynucleotide; and

(ii) the second section is not capable of hybridising to the target polynucleotide; said second section comprising a stem-loop structure comprising a first portion, a second portion and a third portion and wherein the second portion is located between the first and third portions, and the first and third portions are complementary to each other, wherein the stem-loop structure is not labelled with a detectable label;

and further wherein the oligonucleotide is (a) not cleaved by a 3'-5' exonuclease and (b) not extended by a polymerase, when used in a method of detecting a target polynucleotide.

The second portion of the second section (the loop) serves to enable the 360° folding back together of the first and third portions (the stem sequences). 3' stem-loop structures are used by the oligonucleotides of this invention to discourage the pairing with other oligonucleotides in PCR or amplification mixes (in particular multiplex formulations with multiple primer sequences present) and to prevent 3'-5' exonuclease cleavage of 3' PCR blocking modifications. The 3' stem-loop structures are also used to prevent 3'-5' exonuclease cleavage that occurs independently of oligonucleotide dimer formation, e.g. when probes hybridise to target nucleic acid during amplification. The 3' stem-loop structures avoid probe extension and the generation of additional higher $T_m$ melting peaks that can prevent detection of amplified target sequences. The stem-loop sequences of the invention are attached to the 3' ends of probe oligonucleotides, optionally via linker or spacer modifications, and do not hybridise to amplified nucleic acid target sequences.

Stem-loop structures possess two nucleotide sequences that have considerable complementarity to each other separated by a non-complementary sequence. The complementary sequences form a double-stranded "stem" through intramolecular self-hybridisation and the intervening non-complementary sequence creates a "loop". DNA stem-loop secondary structures are also commonly referred to as "hairpins".

The stem-loop sequences should preferably form hairpin structures during the extension phase of PCR to prevent removal of the 3' probe modification by 3'-5' exonuclease activity. The extension phases of 3-step and 2-step PCR protocols are typically between 55°C and 72°C. The melting temperatures of hairpins should, therefore, be greater than 55°C and are preferably greater than 65°C. Suitable melting temperatures can be achieved through intramolecular hybridisation of short stem sequences that are GC-rich. The intervening loop is preferably AT-rich but can comprise any combination of nucleotides as long as the sequence doesn't share homology with the stem, probe or target sequence. The oligonucleotide portions making up the stem of the stem-loop should generally be a GC-rich complementary sequence

When designing oligonucleotide probes, melting temperatures are determined using nearest neighbour thermodynamic calculations (Breslauer et al. 1986; SantaLucia, 1998). Probe melting temperatures represent the stability of intermolecular hybridisation to target DNA sequences. It is also possible to use nearest-neighbour thermodynamics to determine the intramolecular stability of oligonucleotide secondary structures.

Oligonucleotide ΔG (Gibbs free energy) is defined as the net exchange of energy between the system and environment.

The following calculation can be used to determine oligonucleotide ΔG:

$$\Delta G = \Delta H - T.\Delta S$$

Where ΔH is the enthalpy (total energy exchange between the system and environment), ΔS = entropy (the energy spent by the system to organise itself) and T = temperature (degrees Kelvin). For oligonucleotides, positive ΔG values indicate that the system will move in the direction of the single strand, whereas negative ΔG values indicate that the system will move in the direction of the product (i.e. probe/target duplex or oligonucleotide secondary structure). The system is in equilibrium when ΔG = 0 kcal/mole and this is generally the $T_m$ of the system where 50% of the oligonucleotide is single-stranded and 50% is hybridised. Negative ΔG values provide an indication of oligonucleotide secondary structure and the magnitude can determine the performance of primers and probes.

The ability of oligonucleotide sequences to form stem-loop structures can be evaluated using the mfold™ program (Zuker, 2003). The ΔG and $T_m$ values for secondary structures can also be determined with mfold calculated using free energies based on nearest-neighbour thermodynamics (SantaLucia, 1998). The stability of hairpin structures depends on the length and sequence of the stem and loop components (see Table 6 of examples).

The oligonucleotides of the probe system have a sequence complementary to a target polynucleotide sequence. Thus, the oligonucleotide is capable of hybridising to the target polynucleotide sequence under appropriate conditions. Thus, unless the context indicates otherwise, by "complementary" we include the meaning that the oligonucleotide is able to hybridise to a target polynucleotide sequence. The oligonucleotide may be fully complementary to the target polynucleotide sequence (i.e. there is a perfect match in terms of base pairing between the oligonucleotide), or the oligonucleotide may be partially complementary to the target polynucleotide sequence (i.e. there are one or more mismatches between the oligonucleotide and the target polynucleotide sequence, but the oligonucleotide is still able to hybridise). Typically, when the oligonucleotide is partially complementary with the target polynucleotide, there are fewer than 5 mismatches, preferably 1 or 2 or 3 or 4 mismatches, more preferably one mismatch.

Where reference is made to "hybridisation" or the ability of an oligonucleotide and/or primer to "hybridise" to another nucleotide sequence, the skilled person will understand that such hybridisation is capable of occurring under conditions used for melting or annealing curve analysis, typically performed between 15°C and 95°C, preferably between 35°C and 75°C.

The first (5') two nucleotides of the stem (and/or linker) sequence should not be complementary to the target, to avoid increasing the length of probe hybridisation. Preferably the entire 5' stem (and/or linker) sequence should not be complementary to target nucleotides to avoid increasing the length of probe hybridisation. Furthermore, the sequence of the linker, stem and loop sequences should not be complementary to the probing sequence, where three or less consecutive nucleotides should be complementary to ensure formation of the hairpin and correct probe hybridisation to nucleic acid targets.

The target polynucleotide may be any polynucleotide or sequence variation therein of interest. The target polynucleotide may therefore be derived from any source, depending on the application for which the detection is being performed, where such sources include organisms that comprise nucleic acids, i.e. viruses; prokaryotes, e.g. bacteria, archaea and cyanobacteria; and eukaryotes, e.g. members of the kingdom protista, such as flagellates, amoebas and their relatives, amoeboid parasites, ciliates and the like; members of the kingdom fungi, such as slime molds, acellular slime molds, cellular slime molds, water molds, true molds, conjugating fungi, sac fungi, club fungi, imperfect fungi and the like; plants, such as algae, mosses, liverworts, hornworts, club mosses, horsetails, ferns, gymnosperms and flowering plants, both monocots and dicots; and animals, including sponges, members of the phylum cnidaria, e.g. jelly fish, corals and the like, combjellies, worms, rotifers, roundworms, annelids, molluscs, arthropods, echinoderms, acorn worms, and vertebrates, including reptiles, fishes, birds, snakes, and mammals, e.g. rodents, primates, including humans. In some embodiments, the target polynucleotide may be from a synthetic source.

As used herein, "nucleic acid" means either DNA, RNA, single-stranded or double-stranded, and any chemical modifications thereof. Modifications include, but are not limited to, those which provide other chemical groups that incorporate additional charge, polarisability, hydrogen bonding, electrostatic interaction, and functionality to the nucleic acid. Such modifications include, but are not limited to, 2'-position sugar modifications, 5-position pyrimidine modifications, 8-position purine modifications, modifications at exocyclic amines, substitution of 4-thiouridine, substitution of 5-bromo or 5-iodo-uracil; backbone modifications, methylations, unusual base-pairing combinations such as the isobases isocytidine and isoguanidine and the like. Modifications can also include 3' and 5' modifications such as capping.

As used herein, a detectable label is any label that may be detected by any means. For example, the label may be visually detectable, chemically detectable, or physically detectable.

In a second aspect of the invention there is provided a method of detecting the presence of a target polynucleotide and/or sequence variations within the target polynucleotide in a sample of interest, comprising the steps of:

(i) providing a single stranded oligonucleotide having a 5' end and a 3' end, said oligonucleotide comprising a first and second section, the first section being positioned 5' of the second section; and wherein:

the first section is labelled internally with at least two detectable labels, wherein the at least two detectable labels are the same, and is capable of hybridising to a target polynucleotide; and

the second section is not capable of hybridising to a target polynucleotide; said second section comprising a stem-loop structure comprising a first portion, a second portion and a third portion and wherein the second portion is located between the first and third portions, and the first and third portions are complementary to each other, and wherein the stem-loop structure is not labelled with a detectable label;

(ii) exposing the sample of interest to the oligonucleotide of (i);
(i) detecting a change in the detectable label, wherein a change in the detectable label indicates the presence of the target polynucleotide and/or the sequence variations within the target polynucleotide; and wherein there is an absence of (a) cleavage of the oligonucleotide by a 3'-5' exonuclease and (b) extension of the oligonucleotide by a polymerase when the method is performed.

The "Sample of interest" may be any sample derived from any source and includes both *in vitro* and *in vivo* samples. The sample may be directly derived from a living organism, such as tissue, cell or blood sample or may alternatively be an environmental sample that may or may not comprise at least one organism (either dead or alive) for example a microorganism.

By "3'-5' exonuclease" we include specific exonuclease enzymes as well as other molecules possessing 3'-5' exonuclease activity in addition to their primary function, e.g. DNA polymerase. For example, a number of DNA polymerase enzymes are specifically designed to include active 3'-5' exonuclease activity because this imposes sequence fidelity (i.e. removal of mismatched DNA elongation sequence compared to the target template sequence). These enzymes can also have advantageous high processivity so that the amplification process is rapid. Examples of such enzymes are Platinum Taq High Fidelity™ (Invitrogen), Phusion Hot Start High Fidelity DNA polymerase™ (Thermo Scientific), Q5 Hot Start High Fidelity DNA polymerase™ (New England Biolabs), AccuPrime DNA polymerase High Fidelity™ (Invitrogen), Hot Start Hi Fidelity™ (Qiagen) and AccuTaq LA DNA polymerase™ (Sigma Aldrich).

In respect of both the oligonucleotide of the first aspect and the method of the second aspect the following features apply to the oligonucleotide per se and/or the oligonucleotide used as part of the method.

In one embodiment, the oligonucleotide only includes one stem-loop structure i.e. that which is present in the second section of the oligonucleotide, and as such there is no stem-loop structure in the first section. In a further embodiment, there are no secondary structures in the first section of the oligonucleotide.

In one embodiment the first section of the oligonucleotide is labelled internally with the at least two detectable labels. It is preferable if the detectable labels are present without an associated quencher. It is also preferable if the at least two detectable labels on any given oligonucleotide of the invention are the same.

In a preferred embodiment, the detectable label is a visually detectable label. As used herein, the term visually detectable label includes any label that excites anywhere in the electromagnetic spectrum (including non-visible and visible portions of the electromagnetic spectrum). Visually detectable labels also include labels which may be visualised indirectly, such as by radiolabels causing a visual change in a radio-sensitive material (e.g. film). Examples of visually detectable methodologies include manual visualisation of a fluorophore signal in the visible light spectrum, near-infrared fluorescence (such as those sold by Lumiprobe™ - see http://www.lumiprobe.com/c/nir-infrared-dyes?gclid=COvg3ZiEjLkCFYOWtAodXSUA_g), surface-enhanced Raman probes™ (for example Sun (2007)) and DNA sensor detection using modulation of ionic conductance (for example, Wang (2009)).
Conveniently the second section of the oligonucleotide is not capable of hybridising to the first section of the oligonucleotide.
It is preferred that the stem-loop structure does not disassociate when the oligonucleotide hybridises to the target polynucleotide.
The first section of the oligonucleotide may be between 15 and 40 or between 18 and 30 nucleotide residues in length; and/or the second section of the oligonucleotide may be between 7 and 40 or between 12 and 22 nucleotide residues in length.

The length of the oligonucleotides are preferably such that it is suitable for hybridising with the target polynucleotide, to provide a stable hybrid whose melting temperature depends on the exact sequence of the target. Oligonucleotides containing less than 15 nucleotide residues in many cases do not form sufficiently stable hybrids in temperatures ranging between 35°C and 70°C, particularly where the two hybridising sequences are not fully complementary, although they can be used in some circumstances. Linear oligonucleotides, which are longer than about 30 nucleotide residues may form hybrids whose melting temperature is relatively insensitive to the possible presence of a single nucleotide mismatch, although they can be used in some circumstances.

Each of the first and third portions of the stem-loop structure may comprise between 2 and 10 nucleotides or between 4 and 8 nucleotides. The first and third portions do not need to include an identical number of nucleotides. The second portion of the stem-loop structure may comprise between 3 and 20 nucleotides or between 4 and 6 nucleotides.

The oligonucleotide may also be modified at the 3' end with at least one modification to prevent extension by a polymerase and/or dimer formation, for example on the amplifying target nucleic acid or through partial homology with primer sequences so as to create a probe-primer pairing suitable for priming a DNA polymerase strand extension during the amplification reaction. The use of 3' blocking moieties in PCR reactions is described in (French *et al.* 2001, Ben Gaied *et al.* 2010). 3' modifications include but are not limited to 3' phosphate, 3' propanol, 3' amino C7, 3'-spacer C3 CPG, hexanedial, 2',3'-dideoxynucleoside, 3'-deoxynucleoside CPG, 3' biotin pyrene dU and 3' pyrene dT. Multiple 3' modifications may be used to enhance the ability to prevent PCR extension, for example combining 3' propanol with hexaethylene glycol (HEG) or pyrene dU combined with spacer C3.

The stem-loop structure of the oligonucleotide acts to prevent (i) cleavage of the oligonucleotide by a 3'-5' exonuclease and/or (ii) extension of the oligonucleotide by a polymerase. This activity (as well as any additional 3' modification that is present) preferably results in improved detection of nucleic acid target sequences in comparison to a control method utilising an oligonucleotide without the second section.

In one embodiment, the first section and the second section of the oligonucleotide are separated by a linker or spacer modification. A linker or spacer modification can be used to permit hairpin formation without causing interference for probe hybridisation to target nucleic acids. A linker may be formed of nucleic acids and may comprise 1-20 nucleotides, typically 1-10 nucleotides, preferably 1-5 nucleotides. Suitable spacer modifications include but are not limited to C3 spacer phosphoramidites, dSpacer CE phosphoramidite (1'2'-dideoxyribose), pyrrolidine-CE phosphoramidite, hexanediol, triethylene glycol and hexaethylene glycol.

Preferably, the melting temperature ($T_m$) of the second oligonucleotide section is greater than 55°C, for example between 55°C and 95°C or between 65°C and 95°C, or between 80°C and 95°C.

The melting temperature ($T_m$) of an oligonucleotide is the temperature in °C at which 50% of the molecules in a population of a single-stranded oligonucleotide are hybridised to their complementary sequence and 50% of the molecules in the population are not-hybridised to said complementary sequence. The $T_m$ may be determined empirically, for example $T_m$ may be measured using melting or annealing curve analysis, e.g. using a Bio-Rad CFX instrument on a 96-well white plate. The $T_m$ of an oligonucleotide probe is the temperature point of greatest rate of change of fluorescence with temperature between the hybridised and non-hybridised states on the probe. Melting peaks may be generated from melting curve data by (-dF/dT). The melting temperature is fundamentally determined by the temperature of a solution containing the oligonucleotides being slowly raised, while continuously observing a fluorescence signal, in order to construct a graph of the negative derivative of fluorescence signal intensity with respect to temperature (-dF/dT) against temperature. The melting temperature ($T_m$) of the hybrid appears as a peak, and provides information about the sequence of the polynucleotide target. The $T_m$s generated through melting analysis of the oligonucleotide may be used to distinguish polymorphic targets.

Where reference is made to a $T_m$ for hybridisation involving part of an oligonucleotide, the relevant $T_m$ is considered to be the $T_m$ that can be calculated from a nearest neighbour analysis of the sequence involved.

As an alternative, the analysis may be performed by cooling the solution slowly from high temperature and determining the annealing temperature ($T_a$). The annealing temperature is typically between 30°C and 70°C. The preferred annealing temperatures of oligonucleotides with fully complementary target sequences is between 40°C to 65°C, preferably between 41°C and 65°C, and more preferably between 45°C and 65°C.

Furthermore, the stability of the second oligonucleotide section may be between -1 kcal/mol and -10 kcal/mol or between -2 kcal/mol and -6 kcal/mol as calculated using (and as discussed above):

$$\Delta G = \Delta H - T.\Delta S$$

In one embodiment, the visually detectable label is a fluorophore or a dye.

The detectable label is preferably a fluorophore or a dye. Fluorophores of interest include, but are not limited to fluorescein dyes (e.g. fluorescein dT, 5-carboxyfluorescein (5-FAM), 6-carboxyfluorescein (6-FAM), 2',4',1,4,-tetrachlorofluorescein (TET), 2',4',5',7',1,4-hexachlorofluorescein (HEX), and 2',7'-dimethoxy-4',5'-dichloro-6-carboxyfluorescein (JOE)), cyanine dyes such as Cy5™, dansyl derivatives, rhodamine dyes (e.g. tetramethyl-6-carboxyrhodamine (TAMRA), ATTO dyes (such as ATTO 647N) and tetrapropano-6-carboxyrhodamine (ROX)), DABSYL, DABCYL, cyanine, such as Cy3™, anthraquinone, nitrothiazole, and nitroimidazole compounds, or other non-intercalating dyes. Fluorophores of interest are further described in International Patent Applications WO 01/42505 and WO 01/86001.

As used herein, "fluorophore" (also referred to as fluorescent group) refers to a molecule that, when excited with light having a selected wavelength, emits light of a different wavelength. Examples of fluorophore containing oligonucleotides include the HyBeacon II® probes described in WO 2007/010268.

Labelled oligonucleotides may contain modified bases such as phosphorothioate-modified bases. The number of phosphodiester linkages replaced by phosphorothioates in any given oligonucleotide/primer can range from none to all of the phosphodiester bonds being replaced by phosphothioates, for example one, two, three, four or more. The oligonucleotide(s) preferably contain phosphorothioates at, at least one, at least or at least three of the internal bases of the oligonucleotide. In one embodiment the phosphorothioate-modified bases (where there is more than one) are separated by at least one, e.g. one to three, unmodified (phosphorodiester) bases, for example alternate bases within the oligonucleotide(s)/primer(s) may be phosphorothioates. See, for example, PCT/GB2012/050645, for discussion of phosphorothioate incorporation patterns that are considered also to be useful in relation to the present description. Typically the probes of the description exhibit enhanced detectability (such as fluorescence emission) when hybridised with target sequences. However, in principle target sequence detection may be achieved by either an enhancement of detectability (e.g. fluorescent emissions) or by a reduction of a detectability (e.g. quenching of fluorescent emissions). The magnitude of change by enhancement or quenching upon hybridisation is at least 10% higher or lower than the detected signal observed with single-stranded probe, respectively. Preferably the percentage change is greater than 20%. More preferably the percentage change is greater than 50%.

In the case of fluorophore labels, fluorescence enhancement can occur upon target hybridisation when fluorophore-labelled residues are placed in all sequence environments. Placing fluorophore-labelled residues adjacent to G's may result in the highest levels of fluorescence enhancement in the duplex state. However, fluorescence enhancement upon target hybridisation will also occur when fluorophore-labelled residues are located within regions of high C abundance.

All DNA bases are able to quench fluorescence to some extent, where G has the greatest such ability. For the avoidance of doubt, the term "associated quencher" does not include DNA bases which form part of the oligonucleotide. Fluorophores on the oligonucleotides of the description may interact with the bases of single-stranded DNA such that fluorescence is quenched. Gs may modulate fluorescence strength but all dequench significantly on hybridisation. Fluorescence from the internally attached fluorophores of the oligonucleotides is enhanced upon duplex formation irrespective of the location and abundance of guanines in the probe and target strand. Dye-Dye interactions also cause fluorescence quenching in the single-stranded state. Typically probe hybridisation removes these dye-dye interactions causing fluorescence enhancement permitting target detection.

The labelled oligonucleotides emit significantly greater amounts of fluorescence when hybridised to complementary nucleic acid sequences compared with the single-stranded (non-hybridised) conformation despite the absence of a quencher component.

In alternative embodiments, the first section of the oligonucleotide may be labelled with three or more; or four or more detectable labels.

In the second aspect of the invention, method step (iii) is undertaken using either melting curve analysis or annealing curve analysis in order to analyse the change in the detectable label.

Optimisation of the lengths and $T_m$s of oligonucleotides is performed by nearest-neighbour analysis to design probes with $T_m$s between 30°C and 75°C, preferably between 50°C and 65°C when hybridised to fully complementary target sequences.

In one embodiment, the methods may be used to detect the presence of specific known polymorphisms. For example, known polymorphisms that are indicative of the presence of a particular phenotype, disease state, disease susceptibility, predicted response to medication or specific strains of micro-organisms. The known polymorphisms may be detected conducting a melting or annealing analysis which generates a defined melting peak $T_m$ or a defined annealing peak $T_a$ that may be cross referenced to identify the presence of the known polymorphisms. The methods may therefore be used in various fields including but not limited to diagnostics, forensic science, paternity and relationship testing, linkage mapping, microbial typing or traceability within the food chain.

The methods of the description are not limited to detecting known polymorphisms and may be used to detect an unknown polymorphism. This may be achieved by generating a previously unknown melting peak $T_m$ or annealing peak $T_a$.

The methods of the description may be used in target detection, SNP genotyping, or detection of length polymorphisms and repetitive sequences (French et al. 2001, French et al. 2002, French et al. 2007, French et al. 2008).

In one embodiment of the method, the detection step is used in target detection, SNP genotyping, or detection of length polymorphisms and repetitive sequences.

Polynucleotide targets which may be identified using the methods of the description include any nucleic acid-containing targets, such as native DNA or RNA. The nucleic acids may where appropriate include sequences that include any of the known base analogs of DNA and RNA such as 4 acetylcytosine, 8-hydroxy-N6-methyladenosine, aziridinylcytosine, pseudoisocytosine, 5-(carboxyhydroxyl-methyl) uracil, 5-fluorouracil, 5-bromouracil, 5-carboxymethylaminomethyl-2-thiouracil, 5-carboxymethyl-aminomethyluracil, dihydrouracil, inosine, N6-isopentenyladenine, 1-methyladenine, 1-methylpseudo-uracil, 1-methylguanine, 1-methylinosine, 2,2-dimethyl-guanine, 2-methyladenine, 2-methylguanine, 3-methyl-cytosine, 5-methylcytosine, N6-methyladenine, 7-methylguanine, 5-methylaminomethyluracil, 5-methoxy-amino-methyl-2-thiouracil, beta-D-mannosylqueosine, 5'-methoxycarbonylmethyluracil, 5-methoxyuracil, 2-methylthio-N- isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine and 2,6-diaminopurine; or they may contain PNAs.

The oligonucleotides used in the method of the description may include such base analogs or PNAs as appropriate, though this may not be typical.

The method of the description may also be used in conjunction with an 'isothermal' methodology of nucleic amplification such as a Loop-mediated isothermal amplification (LAMP) method, wherein a section of the probe constitutes a LAMP primer (see Notomi *et al.* (2000)).

In a third aspect of the invention there is provided a use of an oligonucleotide as defined in the first aspect in a method for detecting the presence of a target polynucleotide and/or sequence variations within the target polynucleotide in a sample of interest.

The use may be for target detection, SNP genotyping, or detection of length polymorphisms and repetitive sequences.

In a fourth aspect of the invention there is provided a method of making an oligonucleotide as defined in the first aspect, comprising the steps of:

(a) providing a first oligonucleotide labelled with at least two detectable labels and is capable of hybridising to a target polynucleotide;

(b) providing a second oligonucleotide which is not capable of hybridising to a target polynucleotide; said second oligonucleotide being capable of forming a stem-loop structure comprising a first portion, a second portion and a third portion and wherein the second portion is located between the first and third portions, and the first and third portions are complementary to each other;

(c) ligating the first and second oligonucleotides to form a single oligonucleotide, with the first oligonucleotide being positioned 5' of the second oligonucleotide.

Ligation of two oligonucleotides can be conducted using any method of the art to join two single stranded oligonucleotides to form a single oligonucleotide. For example, the two oligonucleotide sections may be clicked together in a ring-strain promoted alkyne-azide cycloaddition reaction (SPAAC reaction) simply by mixing the two oligonucleotides in aqueous buffer. Alternatively, the azide can be located on one of the oligonucleotides to be joined together and the cycloalkyne can be on the other oligonucleotide to be joined together (see Shelbourne (2012); Gerrard (2012); Shelbourne (2011)). As an alternative to the SPAAC reaction, the CuAAC reaction (copper-catalysed alkyne-azide cycloaddition reaction) can be used, in which the cycloalkyne is replaced by a terminal alkyne and the reaction is catalysed by Cu(I) (see El-Sagheer (2012) (2011a) (2009) and Kumar (2007)). Another alternative is to use the Diels-Alder reaction to ligate the two oligonucleotides in which one oligonucleotide is labelled with a diene and the other with a dienophile (see El-Sagheer (2011b)). The above click reactions between two oligonucleotides can be assisted by a complementary oligonucleotide splint to improve the efficiency and rate of the reaction, but this is not essential.

The first and second oligonucleotide sections can be produced by any method of synthesising oligonucleotides. Oligonucleotide synthesis typically proceeds by the stepwise addition of phosphoramidite activated nucleotides or other phosphorus derivatives to a growing oligonucleotide chain on a cleavable solid support. This process continues until the complete oligonucleotide is assembled and is then cleaved from the solid support. Oligonucleotide synthesis is conventionally carried out in the 3' to 5' direction rather than the 5' to 3' direction due to the fact that the requisite monomers are easier to synthesise, but both directions of synthesis are possible. The process described below applies to both directions of oligonucleotide synthesis on solid support. It is a method by which large numbers of oligonucleotides can be synthesised in two or more stages (large scale followed by small scale) using the differing scales to achieve benefits in cost.

The synthesis of oligonucleotides on a large scale (e.g. 15 micromole or greater) allows highly efficient oligonucleotide synthesisers to be used with optimised synthesis cycling conditions and larger economies of scale resulting from more efficient use of reagents and solvents. The large scale synthesis of an oligonucleotide containing expensive modifications such as dye labelled nucleobases or protected aminoalkyl, alkyne or cycloalkyne-labelled nucleobases (either the 5'-portion or the 3'-portion of the oligonucleotide) is particularly cost-effective. The oligonucleotide that has been assembled in the initial large scale synthesis is left attached to the solid support, with all its nucleobase, phosphate and terminal hydroxyl protecting groups attached. The solid support is then subdivided into small portions and further oligonucleotide synthesis can be performed on each portion separately. After assembly and prior to (or post) the subdivision stage the "large scale" oligonucleotide attached to the solid support can (if required) be modified by methods including but not limited to amide formation, click chemistry, Diels-Alder and maleimide labelling, giving rise to a number of potential modified end products. This method can be used to add fluorescent dyes, haptens, SERS labels or other reporter groups or ligands. Labelling the oligonucleotide whilst on the solid support allows anhydrous solvents to be used, increasing the life time of some labelling reagents (e.g. active esters, maleimides) during labelling, thereby improving the efficiency of the labelling reaction. Furthermore, using large scales and highly efficient large scale synthesisers such as the ÄKTA OligoPilot™ (GE Healthcare) with recirculating reagents in the labelling procedure facilitates efficient use of reagents.

Once the solid support has been divided and any labelling modification (if required) has been performed, the individual smaller quantities of solid support can be treated individually, and subjected to further rounds of oligonucleotide synthesis. Each individual batch can then have a different sequence assembled onto it (the variable target specific element) and this second phase of synthesis can be done on a variety of scales from 1.0 micromole down to 10 nanomole or smaller. Many different small scale DNA synthesisers can be used for this second phase of oligonucleotide synthesis (e.g. ABI 3400™, ABI 3900™, ABI 394™, PerSeptive Expedite™, Dr Oligo 96/193™, Bioautomation MerMade™). These subsequent smaller syntheses can then be deprotected using standard conditions giving small scale products at very low cost. This process is as efficient as standard single phase oligonucleotide synthesis, yielding high quality oligonucleotides. Oligonucleotide purification by gel-electrophoresis or HPLC is unnecessary for applications using probes (such as those of the description) synthesised by this method because of high synthesis quality.

In a fifth aspect of the invention there is provided an oligonucleotide library comprising a plurality of oligonucleotides as defined in the first aspect.

In one embodiment, the plurality of oligonucleotides may each comprise a different detectable label. The plurality of oligonucleotides are attached to a solid support.

In a sixth aspect of the invention there is provided a kit of parts comprising:

(a) an oligonucleotide as defined in the first aspect; and
(b) instructions for use.

An alternative kit of parts may comprise:

(a) the first section of an oligonucleotide as defined in the first aspect;
(b) the second section of an oligonucleotide as defined in the first aspect;
(c) instructions for use.

The alternative kit may further comprise means for conjugating the first and second sections to form a single oligonucleotide. Such means include the reagents and apparatus for performing two phase oligonucleotide synthesis and click chemistry methods as discussed in relation to the fourth aspect.

The kits of the description may also comprise one or more selected from reaction buffer (for PCR or isothermal amplification), dNTPs, oligonucleotide primers, enzyme and further additives including but not limited to $MgCl_2$, Bovine Serum Albumin (BSA), Dimethyl Sulfoxide (DMSO), Betaine, Tween-20 and carrier RNA.

Kits may be provided in a liquid form or as stabilised reagents (using a technique including but limited to lyophilisation and gelification)

The present description provides a novel oligonucleotide probe modification that is explained in more detail in the following description and examples.

## Examples and Figures

The invention will now be described in more detail with reference to the following figures and examples.

The invention is demonstrated through a number of examples. These show:

(i) The results of DNA polymerase 3'-5' exonuclease activity which causes PCR extension of probe oligonucleotides and the generation of peaks of increased melting temperature ($T_m$), where $T_m$s are greater than expected for full length probe hybridisation.
(ii) The reduction of this probe extension using different 3' cap modifications and polymerases.
(iii) Prevention of probe extension using stem-loop sequences attached to the 3' ends of oligonucleotides.
(iv) The generation and prevention of probe extension using melting and annealing curve analysis.

*Figure Legends*

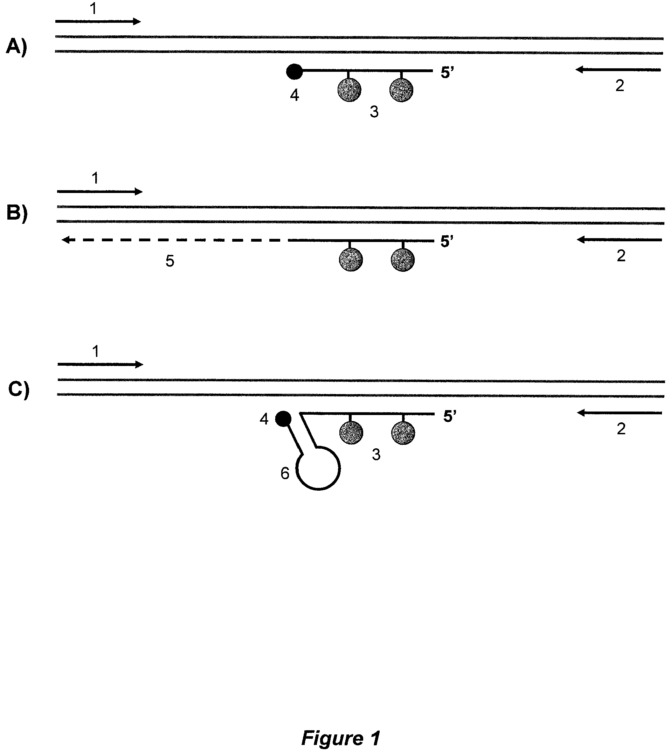

**Figure 1 - Schematic representation of oligonucleotides**

A) Detection of amplified target sequences using a standard HyBeacon probe (WO 01/73118, WO 07/010268).
B) The 3'-5' exonuclease activity of DNA polymerase removes the 3' PCR blocking modification allowing extension of the HyBeacon probe and amplification of a truncated target.
C) An oligonucleotide with a 3' stem-loop structure is used to prevent 3'-5' exonuclease removal of the 3' modification and avoid probe extension.

Figure illustrations present a forward excess primer (1), reverse limiting primer (2), dual-labelled HyBeacon probe (3), 3' PCR blocking modification (4), extension from an unblocked probe (5) and a 3' stem-loop sequence to prevent probe extension.

**Figure 2: Multiplex amplification of P2 and G1 sequences using Phire polymerase** and target detection using A) P2-FL and B) G1-TxR probes. Target detection and probe extension peaks are indicated.

**Figure 3: Multiplex amplification of P2 and G1 sequences using the Q5 enzyme** and target detection using A) P2-FL and B) G1-TxR probes. Target detection and probe extension peaks are indicated.

**Figure 4: Melting peak analysis using the P2-PYR probe** in multiplex PCR formulations containing A) Phire and B) Q5 enzymes. Target detection and probe extension peaks are indicated.

**Figure 5: Melting peak analysis using the P2-3SL probe** in multiplex PCR formulations containing A) Phire and B) Q5 enzymes. The 3' stem-loop sequence clearly prevents probe extension and generation of the higher $T_m$ melting peak.

**Figure 6: Melting peak analysis using the P2-3SLPYR probe** in multiplex PCR formulations containing A) Phire and B) Q5 enzymes. The 3' stem-loop sequence clearly prevents probe extension and generation of the higher $T_m$ melting peak.

**Figure 7: Melting peak analysis using the P2-14nt probe** in multiplex PCR formulations containing A) Phire and B) Q5 enzymes. The 3' stem-loop sequence prevents probe extension with Phire polymerase but the higher $T_m$ extension peak is still observed with the Q5 enzyme.

**Figure 8: Melting peak analysis using the P2-2nt probe** in multiplex PCR formulations containing A) Phire and B) Q5 enzymes. Two nucleotides of unhybridised sequence added to the 3' end of the probe are not sufficient to prevent probe extension.

**Figure 9: Melting curve analysis performed with A) P2-5SL and B) P2-IL probes** having 5' and internal stem-loop sequences, respectively. Reactions containing target (+ve) and no template controls (NTC) are indicated.

**Figure 10: A) Annealing and B) melting curve analysis of the CXCL12-PYR probe** with target detection and probe extension peaks indicated. Two samples of C/C genotype and two samples of C/T genotype are presented, with only the C allele generating a melting peak in the presence of probe extension.

**Figure 11: A) Annealing and B) melting curve analysis of the CXCL12-3SLPYR probe.** The 3' stem-loop sequence clearly prevents probe extension and generation of the higher $T_m$ melting peak. Peak data demonstrates simultaneous detection of C and T alleles in the absence of probe extension.

**Materials and methods for the Examples**

*Oligonucleotide probe design and synthesis*

Standard DNA phosphoramidites, solid supports and additional reagents were purchased from Link Technologies or Applied Biosystems Ltd. All oligonucleotides were synthesised on an Applied Biosystems 394 automated DNA/RNA synthesiser using a 0.2μmole phosphoramidite cycle of acid-catalysed detritylation, coupling, capping and iodine oxidation. Normal monomers (A, G, C and T) were allowed to couple for 25 seconds and all other monomers for an additional 300 seconds. Stepwise coupling efficiencies and overall yields of monomers with DMT protection were determined by measuring trityl cation conductivity and in all cases were >98.0%. Cleavage of the oligonucleotides from the solid support was carried out in concentrated aqueous ammonia (33%) at 55°C for 5 hours in a sealed tube. Fluorophores were attached to internal residues in the probe sequence using Fluorescein dT (Glen Research, Sterling, VA) or Texas Red attachment using 2' aminoethoxy T (Richardson *et al.* 2010). The oligonucleotides of the examples possess a 3'-phosphate, 3' propanol, 3'-pyrene or other blocking agent intended to prevent *Taq* mediated extension when the probes are incorporated into PCR assays.

Once synthesis of the target specific sequence was complete the oligonucleotide was cleaved from the resin and deprotected using concentrated aqueous ammonia at room temperature for one hour, followed by 5 hours at 55 °C in a sealed tube. Any impurities from synthesis/deprotection were removed and the oligonucleotide desalted using Illustra NAP-10 gel filtration columns (GE Healthcare) according to the manufacturer's instructions. Oligonucleotide probes were purified by HPLC.

The quantity of oligonucleotide obtained from the synthesis was determined by dissolving an aliquot in a specific volume of water and measuring the UV absorbance at 260nm. Concentration was calculated using the UV absorbance of the oligonucleotide and its extinction coefficient at 260nm. The extinction coefficient of the oligonucleotide was calculated from the sum of the individual extinction coefficients of the unmodified and fluorescently labelled nucleosides of which it is composed.

*Polymerase chain reaction*

PCR volumes were typically 20μl, generally comprising 2μl of sample, 1x PCR buffer, 1μM excess primer, 0.222μM limiting primer, 0.4-0.8μl DNA polymerase, 3mM total MgCl$_2$, 1mM dNTPs (GE Healthcare), 30-150ng/μl BSA (Roche Diagnostics) and 100-300nM of oligonucleotide probe. Target specific PCR formulations are described in the examples below.

Homogeneous amplification and detection of targets was performed with a CFX96 Real-Time PCR detection system (Bio-Rad), with melting or annealing curve analysis performed immediately after PCR amplification. Thermal protocols are described in the examples below. Melting and annealing peaks were constructed using the CFX software (Bio-Rad) by plotting the negative derivative of fluorescence with respect to temperature (-dF/dT on the y-axis) against temperature (x-axis).

*Example 1*

Multiplex PCR was performed to simultaneously amplify G1 and P2 target sequences using Phire Hot Start II DNA polymerase™ (Fisher Scientific). Amplified G1

(GCGGACTGCAAGAAGATTGTAAAGAAAACTTTTTCGAAGCTCTTGCTGTTAT
GGAACGTGAAGGCAGTCGCATTATTGATGTAGATCTCAGTGTTTTGAAACAT GCGGTA) and P2

(CATATTACGAGCTTTTTATAAACCTCCCCAACCAAACTCTACAAAAAGAGTTT

CAATCGATCCCCTATAAATCCGCATATATTTTGGCCGC)

targets (*Chlamydia trachomatis*) were detected with the Texas Red labelled G1-TxR and fluorescein-labelled P2-FL probes, respectively (Table 1).

PCR reaction volumes were 20μl, comprising 2μl sample, 1x Phire™ buffer (Fisher Scientific, UK), 1mM dNTPs, 3mM total magnesium chloride, 100ng/μl carrier RNA, 150ng/μl BSA, 1μM G1 forward primer (G1-LFx), 222nM G1 reverse primer (G1-LRx), 1μM P2 forward primer (P2-F), 500nM P2 reverse primer (P2-R2), 150nM G1-TxR probe, 112.5nM P2-FL probe and 0.8μl of Phire Hot Start II DNA polymerase™ (Fisher Scientific, Loughborough, UK). Primer and probe sequences are detailed in Table 1.

PCR amplification was performed using a CFX96™ thermal protocol, where after an initial denaturation step (95°C 15 seconds), targets were amplified using 10 cycles comprising denaturation (95°C 1 second) and annealing/extension (65°C 1 second) followed by 40 cycles comprising denaturation (85°C 1 second) and annealing/extension (65°C 1 second). Melting curve analysis was performed immediately after amplification by briefly denaturing (95°C 30 seconds) and cooling (30°C 30 seconds) samples prior to increasing temperature from 30°C to 95°C in 0.5°C steps. Fluorescence acquisition was performed in fluorescein and Texas Red dye channels for multiplex detection of amplified targets.

Targets were amplified using 200fg (18 genomic copies) of extracted *Chlamydia trachomatis* DNA. The example may also be conducted using targets that were amplified from synthetic DNA sequences constructed using gene synthesis (GenScript, Hong Kong). Synthetic constructs comprised primer and probe regions which were cloned into pUC57 vectors and sequenced for quality control.

**Table 1:** Oligonucleotide and oligonucleotide target sequences, where F, X, 5 and 3 represent fluorescein dT, Texas Red, 5' trimethoxystilbene and 3' propanol, respectively.

| OLIGO | SEQUENCE |
|---|---|
| G1-LFx | GCGGACTGCAAGAAGATTGTAAAGAAAAC |
| G1-LRx | TACCGCATGTTTCAAAACACTGAGATCTAC |
| G1-TxR | 5CTTCACGXTCCAXAACAGCAAGAG3 |
| P2-F | CATATTACGAGCTTTTTATAAACCTCCCCAAC |
| P2-R2 | GCGGCCAAAATATATGCGGATT |
| P2-FL | 5GGATCGATFGAAACFCTTTTTGTAGA3 |

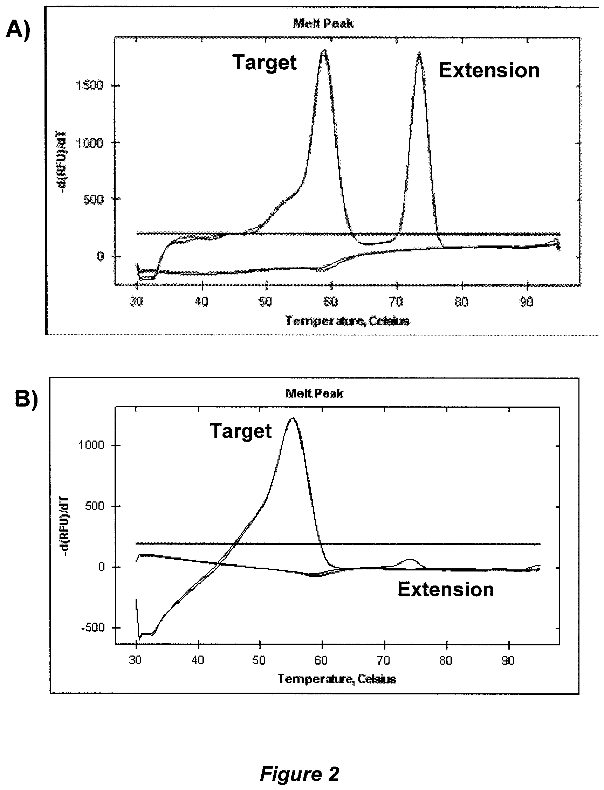

Detection of amplified G1 and P2 target sequences results in melting peaks with $T_m$s of 55.5°C and 59.0°C, respectively (Figure 2).

Probes used 3' propanol modifications to prevent probe extension during PCR amplification. However, an additional higher $T_m$ melting peak was generated using the fluorescein-labelled P2-FL probe caused by removal of the 3' blocking modification (through 3'-5' exonuclease activity) and PCR extension of the oligonucleotide (Figure 1).

Removal of the 3' modification allows the probe to act as a primer, such that a product of 63bp can be generated with the P2-F primer and P2-FL probe. Probe extension incorporates the P2-FL oligonucleotide into the spurious 63bp product thereby generating a higher $T_m$ melting peak than when the intact 26 nucleotide probe hybridises to the 91 bp amplicon. Extension of the P2-FL probe generates a peak at 73.5°C in addition to the P2 target hybridisation peak at 58.5°C (Figure 2A).

Extension of the P2-FL probe potentially occurs through a priming event enabled by the 3'-5' exonuclease activity revealing a terminal sequence with partial homology to another oligonucleotide in the multiplex. The additional 73.5°C was not observed in a singleplex PCR in the absence of G1 oligonucleotides (data not shown). The PrimerList™ tool (Kalendar *et al.* 2001) was used to investigate the potential for dimer formation using the oligonucleotides listed in Table 1. The only obvious oligonucleotide interaction involving the P2-FL probe is a cross-dimer formed with the G1-TxR probe.

A very small extension peak was observed with the G1-TxR probe at 74.0°C using Phire polymerase (Figure 2B).

## *Example 2*

Multiplex amplification of G1 and P2 target sequences was also performed using Q5 Hot Start High-Fidelity DNA polymerase™ (New England Biolabs). The enzyme has an extremely low error rate, reported to be 100-fold lower than *Taq* DNA polymerase. The strong 3'-5' proof reading activity of the Q5 enzyme was tested to examine G1-TxR and P2-FL probe extension.

PCR reaction volumes were 20μl, comprising 2μl sample, 1x Q5 buffer™ (New England Biolabs, Herts, UK), 1mM dNTPs, 3mM total magnesium chloride, 100ng/μl carrier RNA, 150ng/μl BSA, 1μM G1 forward primer (G1-LFx), 222nM G1 reverse primer (G1-LRx), 1μM P2 forward primer (P2-F), 500nM P2 reverse primer (P2-R2), 150nM G1-TxR probe, 112.5nM P2-FL probe and 0.4μl of Q5 Hot Start DNA polymerase™ (New England Biolabs, Herts, UK). Multiplex amplification of targets used the thermal protocol detailed in example 1.

The Q5 enzyme increased the magnitude of the 74.0°C extension peak observed with the P2-FL probe. Probe extension occurs to such an extent with the Q5 enzyme that it almost completely prevents detection of the P2 target sequence

(Figure 3A)

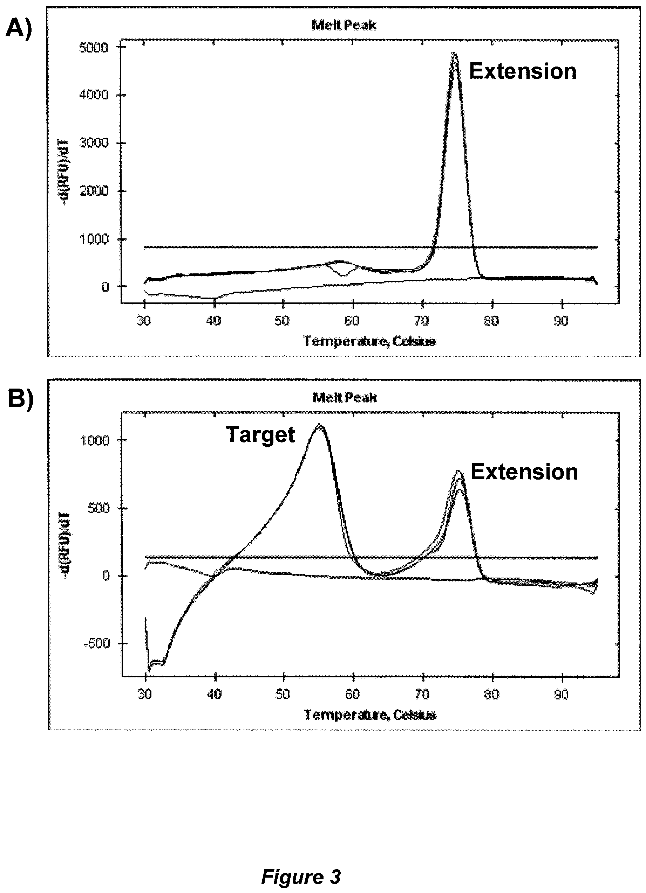

Whilst the Phire™ enzyme of example 1 only generated a small extension peak with the G1-TxR probe, The Q5 enzyme generated a large peak at 75.5°C with the G1-TxR probe (Figure 3A) caused by 3'-5' exonuclease cleavage of the 3' propanol modification and subsequent PCR extension of the oligonucleotide. The Q5 enzyme appears to exhibit more aggressive 3'-5' exonuclease activity compared with Phire increasing the magnitude of the G1-TxR probe extension peak.

*Example 3*

The P2-PYR probe (Table 2) has the same sequence as P2-FL but uses a 3' Pyrene dT Long modification (ATDBio, Southampton, UK) instead of 3' propanol. The P2-PYR probe was used in the multiplex PCR formulations described in Examples 1 and 2 instead of P2-FL.

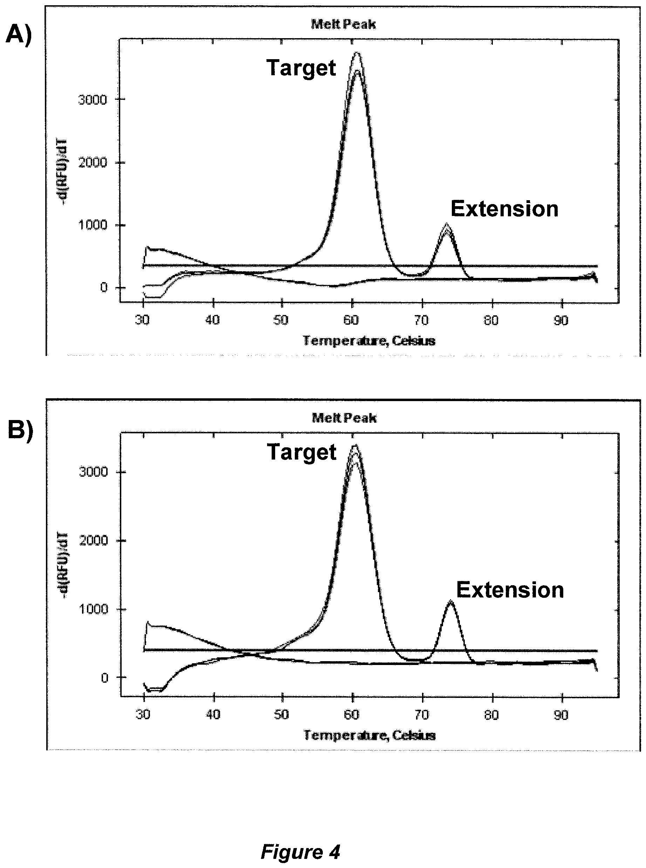

The Pyrene dT modification considerably reduced the formation of the extension products generated with Phire and Q5 enzymes (Figure 4), but the 73.5°C and 74.0°C melting peaks were still observed along with target specific probe hybridisation peaks. The P2-PYR probe generated target peaks at slightly higher $T_m$s (60.5°C with Phire™) than the 3' propanol-capped P2-FL oligonucleotide since pyrene dT modifications are known to be stabilising (Ben Gaied *et al.* 2010).

Pyrene dT may be more resilient to 3'-5' exonuclease cleavage but a proportion of the PCR blocker is still removed allowing probe extension (Figure 4).

**Table 2:** Oligonucleotide and oligonucleotide target sequences, where F, 5, 3 and Y represent fluorescein dT, 5' trimethoxystilbene, 3' propanol and 3' Pyrene dT Long, respectively. Lower case nucleotides represent the stem sequences of stem-loop structures.

| OLIGO | SEQUENCE |
| --- | --- |
| P2-PYR | 5GGATCGA**T**FGAAACFCTTTTTGTAGAY |
| P2-3SL | 5GGATCGATFGAAACFCTTTTTGTAGAggtgTTTTTTc acc3 |
| P2-3SLPYR | 5GGATCGATFGAAACFCTTTTTGTAGAggtgTTTTTTc accY |

*Example 4*

A nucleotide sequence capable of forming a stem-loop structure was added to the 3' end of the P2-FL probe to discourage dimer formation with other oligonucleotides in the multiplex PCR formulation and prevent 3'-5' exonuclease removal of 3' PCR blocking modifications (Figure 1C). The stem-loop structure was formed with the sequence 5'(ggtgTTTTTTcacc)3', where lower and upper case nucleotides form the stem and loop, respectively. This sequence was chosen as one having no homology with the probe sequence, but being capable of achieving a stable stem-loop structure (see Example 9). The P2-3SL and P2-3SLPYR oligonucleotides possess 3' propanol and 3' Pyrene dT Long modifications to prevent PCR extension of probes (Table 2). These probes were used in the multiplex PCR formulations described in examples 1 and 2 replacing the P2-FL oligonucleotide.

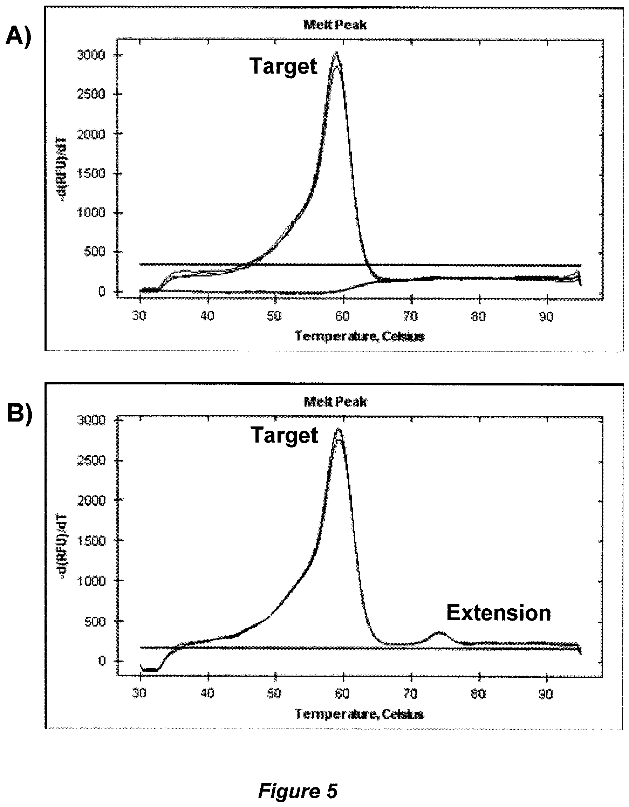

With Phire™ polymerase, the P2-3SL oligonucleotide only generated the target specific hybridisation peak, with no evidence of probe extension observed (Figure 5A). Hybridisation of the P2-3SL probe to amplified targets generated peaks at the same melting temperatures (59.0°C) as the P2-FL probe which lacks the 3' stem-loop structure, indicating that the 5'(ggtgTTTTTTcacc)3' hairpin is not destabilising probe hybridisation to target oligonucleotides.

With Q5 enzyme™, the 3' stem-loop structure of the P2-3SL oligonucleotide prevented most of the extension previously observed with P2-FL probe. A large target specific hybridisation peak was generated at 59.0°C and a very small extension peak was observed at 74.0°C (Figure 5B).

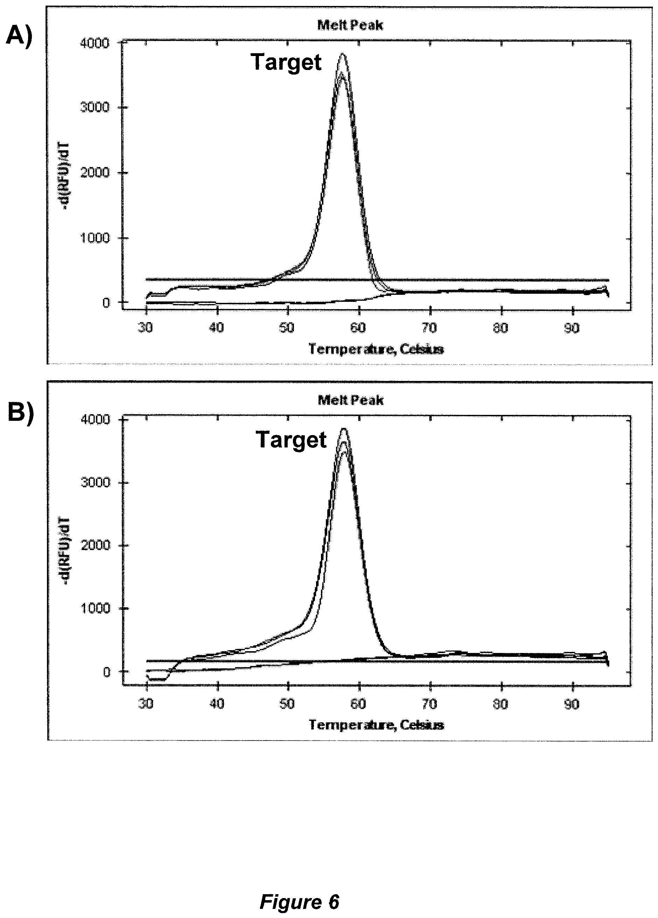

Similarly with Phire™ polymerase, the P2-3SLPYR oligonucleotide only generated the target specific hybridisation peak, with no evidence of probe extension observed (Figure 6A). Hybridisation of the P2-3SLPYR probe to amplified targets generated peaks at 57.5°C. These peaks are 3°C lower than the P2-PYR probe without the stem-loop structure and 1.5°C lower than the P2-FL and P2-3SL oligonucleotides capped with 3' propanol. This suggests that the 3' Pyrene dT Long modification is stabilising the stem-loop structure and consequently destabilising probe hybridisation slightly.

With Q5 enzyme™, the P2-3SLPYR oligonucleotide only generated the target specific hybridisation peak, with no evidence of probe extension observed (Figure 6B). The increased stability of the stem-loop structure provided by the Pyrene dT modification prevented the small amount of probe extension observed with the P2-3SL oligonucleotide.

The 3' stem-loop structure provides protection against 3'-5' exonuclease removal of the PCR blocking modifications to prevent extension of probe oligonucleotides and ensure detection of amplified P2 target sequences. Use of the 3' stem-loop sequence on P2 oligonucleotides does not alter the performance characteristics of the G1-TxR probe in the multiplex, with peaks similar to those presented in Figure 2B and Figure 3B generated with Phire and Q5 enzymes, respectively.

*Example 5*

The oligonucleotide P2-14nt (Table 3) was used to determine whether the benefit provided by the P2-3SL and P2-3SLPYR oligonucleotides was caused by the stem-loop structure or the presence of fourteen unhybridised nucleotides at the 3' end of the probe. The fourteen unhybridised nucleotides lack secondary structure and do not interact with the target binding probe sequence, This probe was used in the multiplex PCR formulations described in examples 1 and 2 replacing the P2-FL oligonucleotide.

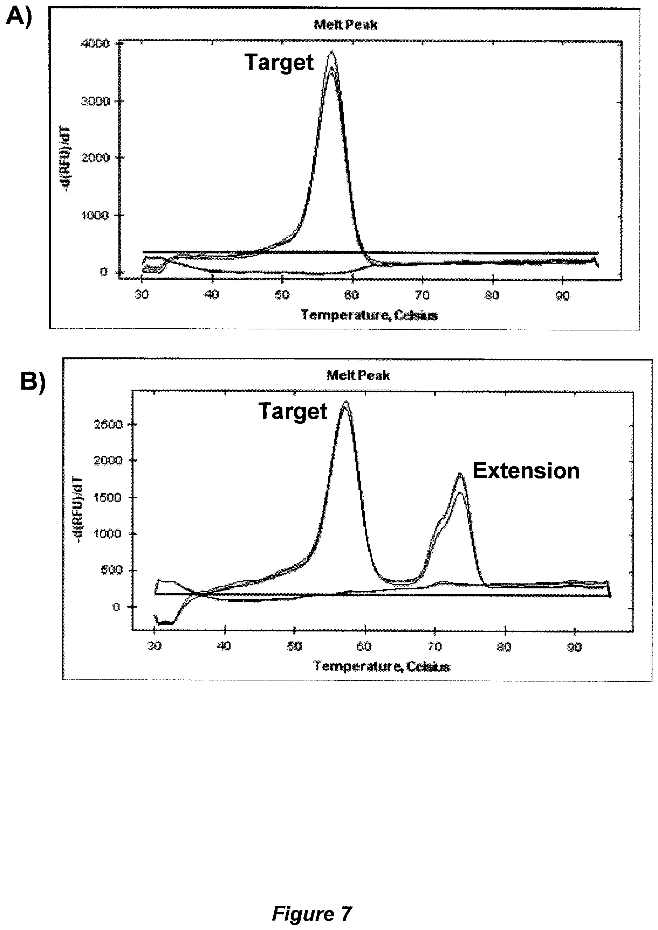

With Phire™ polymerase, the P2-14nt oligonucleotide generated only the target specific hybridisation peak, with no evidence of probe extension observed (Figure 7A). Hybridisation of the P2-14nt probe to amplified targets generated melting peaks with $T_m$s of 57.0°C, indicating that the fourteen base overhang of unhybridised nucleotides was destabilising probe binding.

Whilst the fourteen unhybridised nucleotides were sufficient to prevent probe extension with Phire™, they did not prevent removal of the 3' propanol modification using the Q5 enzyme™. The target specific hybridisation peak and probe extension peak were both observed when the P2-14nt oligonucleotide was used in the multiplex PCR with Q5 Hot Start High-Fidelity DNA polymerase™ (Figure 7B).

The unhybridised sequence at the 3' end of the probe does provide some protection against 3'-5' exonuclease activity, but it does not completely prevent removal of the PCR blocking modification (with the Q5 enzyme). This demonstrates the value of the stem-loop structure described in example 4.

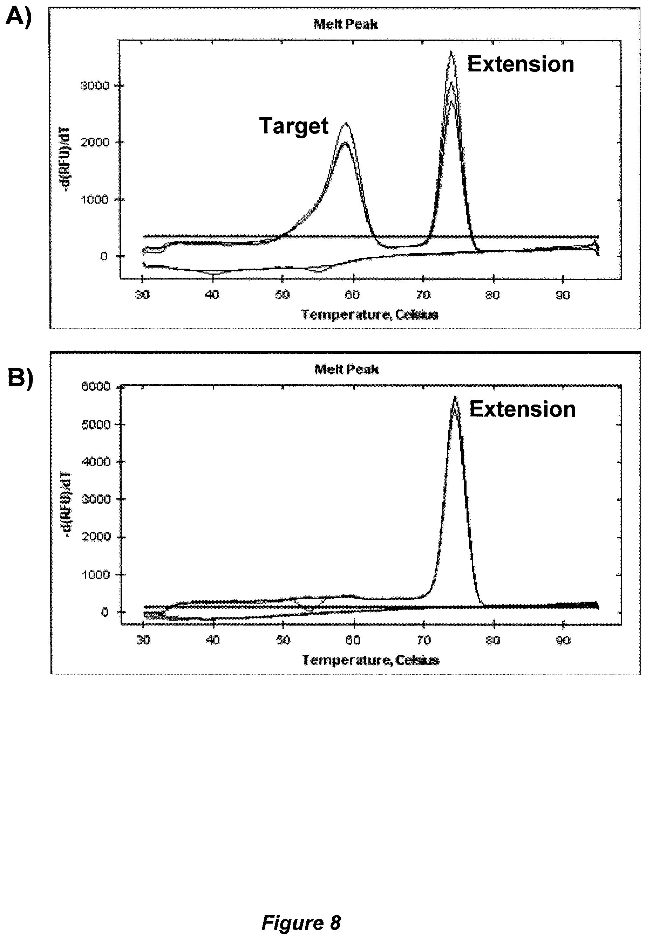

The oligonucleotide P2-2nt (Table 3) has two unhybridised bases at the 3' end of the probe. This did not appear to provide any benefit in the multiplex PCR, producing large probe extension peaks with both Phire™ and Q5™ enzymes (Figure 8).

Table 3: Oligonucleotide and oligonucleotide target sequences, where F and 3 represent fluorescein dT and 3' propanol, respectively. Lower case nucleotides represent unhybridised nucleotides that are not complementary to target sequences.

| OLIGO | SEQUENCE |
|---|---|
| P2-14nt | GGATCGATFGAAACFCTTTTTGTAGAttttttttttcacc3 |
| P2-2nt | GGATCGATFGAAACFCTTTTTGTAGAag3 |

*Example 6*

The oligonucleotides P2-5SL and P2-IL (Table 4) were used to evaluate the performance of probes with 5' and internal stem-loop structures, respectively. These probes were tested in the Q5™ enzyme multiplex described in example 2 replacing the P2-FL oligonucleotide.

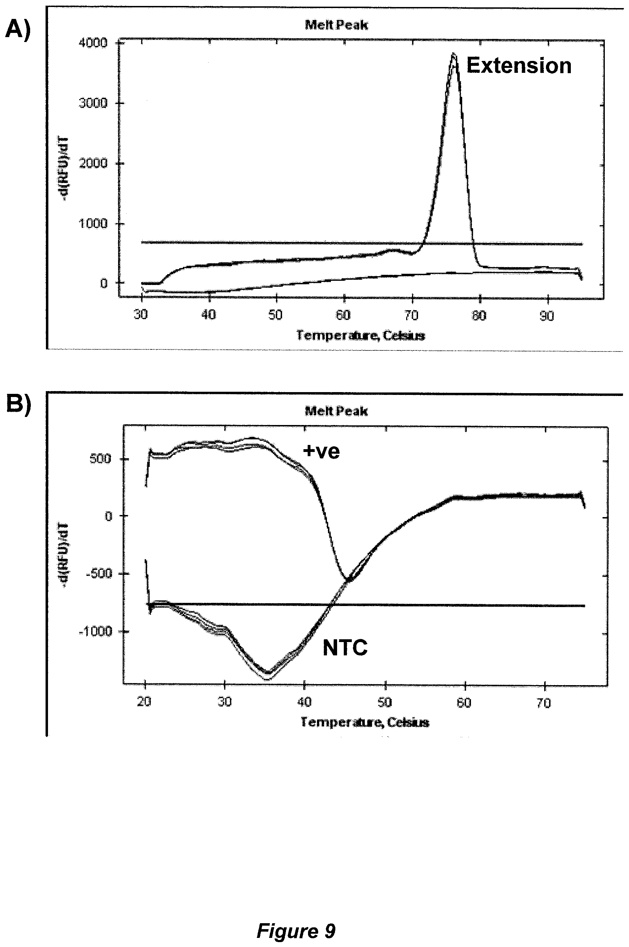

The P2-5SL oligonucleotide generated large extension peaks at 76.0°C, but melting peaks at 59.0°C indicative of target specific probe hybridisation were not observed (Figure 9A). As expected, the 5' stem-loop structure provides no protection against 3'-5' exonuclease removal of the PCR blocking 3' propanol modification. Extension of the probe prevents detection and/or sufficient amplification of the full length P2 target sequence.

The presence of the internal stem-loop in the P2-IL oligonucleotide reduced the stability of probe hybridisation considerably. It also increased the spacing between fluorophore-labelled bases to 19 nucleotides. Obvious peaks were not generated by melting curve analysis but the presence of target could be determined, with positive samples generating melting peak profiles that were clearly different from No templates Controls (NTCs) at low temperatures (Figure 9B). The inclusion of the stem-loop in an 'internal' sequence location appears to be so detrimental to probe-target hybridisation functionality that it is not possible to assess any blocking of 3'-5' exonuclease activity.

Table 4: Oligonucleotide and oligonucleotide target sequences, where F and 3 represent fluorescein dT and 3' propanol, respectively. Lower case nucleotides represent the stem sequences of stem-loop structures.

| OLIGO | SEQUENCE |
|---|---|
| P2-5SL | ggtgTTTTTTcaccGGATCGATFGAAACFCTTTTTGTAG A3 |
| P2-IL | GGATCGATFGAggtgTTTTTTcaccAACFCTTTTTGTAG A3 |

*Example 7*

A polymorphic CXCL12 (human) gene target comprising the rs1746048 C>T SNP was amplified using the CXCL12-F and CXCL12-R primers (Table 5). Amplification of the 88bp target sequence was performed using Phire Hot Start II DNA polymerase™ and Human DNA samples were genotyped with respect to the rs1746048 polymorphism using the fluorescein-labelled HyBeacon™ probe CXCL12-PYR (Table 5).

PCR reaction volumes were 20μl, comprising 2μl sample, 1x Phire™ buffer, 1mM dNTPs, 3mM total magnesium chloride, 30ng/μl BSA, 222nM CXCL12-F forward primer, 1μM CXCL12-R reverse primer, 300nM CXCL12-PYR probe and 0.4μl of Phire Hot Start II DNA polymerase™. Targets were amplified using 2ng of extracted DNA (Human Random Control DNA panel 5, Sigma/ECACC).

PCR amplification was performed using a CFX96™ thermal protocol, where after an initial denaturation step (95°C 1 minute), targets were amplified using 50 cycles comprising denaturation (95°C 5 second) and annealing/extension (65°C 10 seconds). Reactions were denatured immediately after amplification (95°C 30 seconds) prior to performing melting curve or annealing curve analysis. Melting curve analysis comprised a cooling step (35°C 30 seconds) prior to increasing the temperature from 35°C to 85°C in 0.5°C steps. Annealing curve analysis was performed by reducing the temperature from 85°C to 35°C in 0.5°C steps. Fluorescence acquisition was performed in the fluorescein channel of a CFX instrument.

**Table 5:** Oligonucleotide and oligonucleotide target sequences, where F and Y represent fluorescein dT and 3' Pyrene dT Long, respectively. Lower case nucleotides represent the stem sequences of stem-loop structures.

| OLIGO | SEQUENCE |
|---|---|
| CXCL12-F | GATTTCAGGACTGAACAGAGACTGAG |
| CXCL12-R | GTCATGGTAGCTAACTAGAGGTTTCTG |
| CXCL12-PYR | GTGGFAGGATFGAGCGAGTCAGGY |
| CXCL12-3SL | GTGGFAGGATFGAGCGAGTCAGGggccgAAAAcggccY |

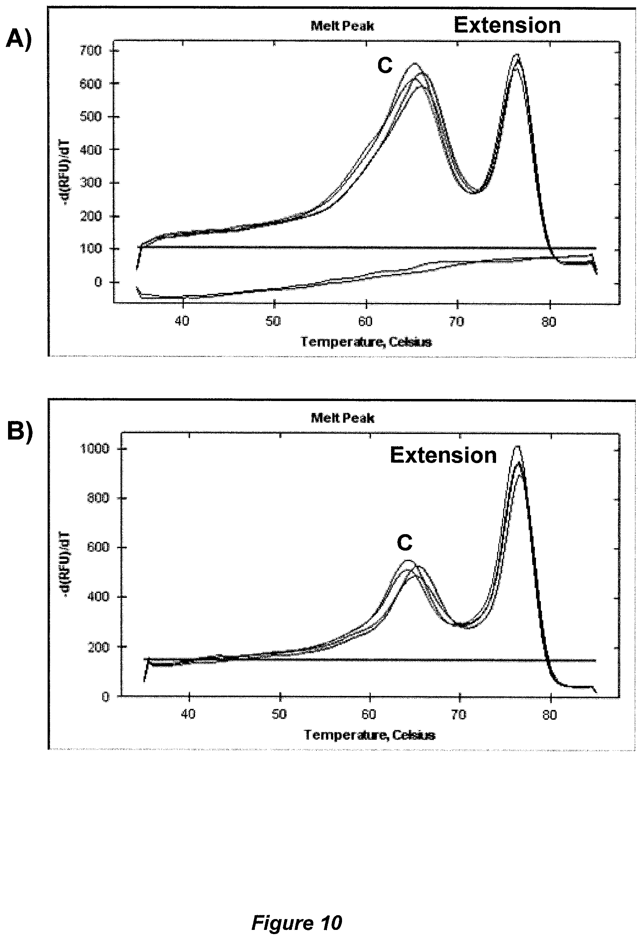

Probe hybridisation to fully complementary target sequences, comprising the C allele of the rs1746048 polymorphism, generates annealing and melting peaks at 65.5°C and 64.5°C (Figure 10A & B respectively). Additional annealing and melting peaks are observed at 76.5°C arising from extension of the CXCL12-PYR probe. Large extension peaks were observed with the CXCL12-PYR even when capped with the 3' pyrene dT Long modification.

Removal of the 3' Pyrene modification through 3'-5' exonuclease activity allows the probe to act as a primer, such that a product of 51 bp can be generated with the CXCL12-R primer and CXCL12-PYR probe. Probe extension incorporates the CXCL12-PYR oligonucleotide into the spurious 51 bp product thereby generating a higher $T_m$ melting peak than when the intact 23 nucleotide probe hybridises to the 88bp amplicon.

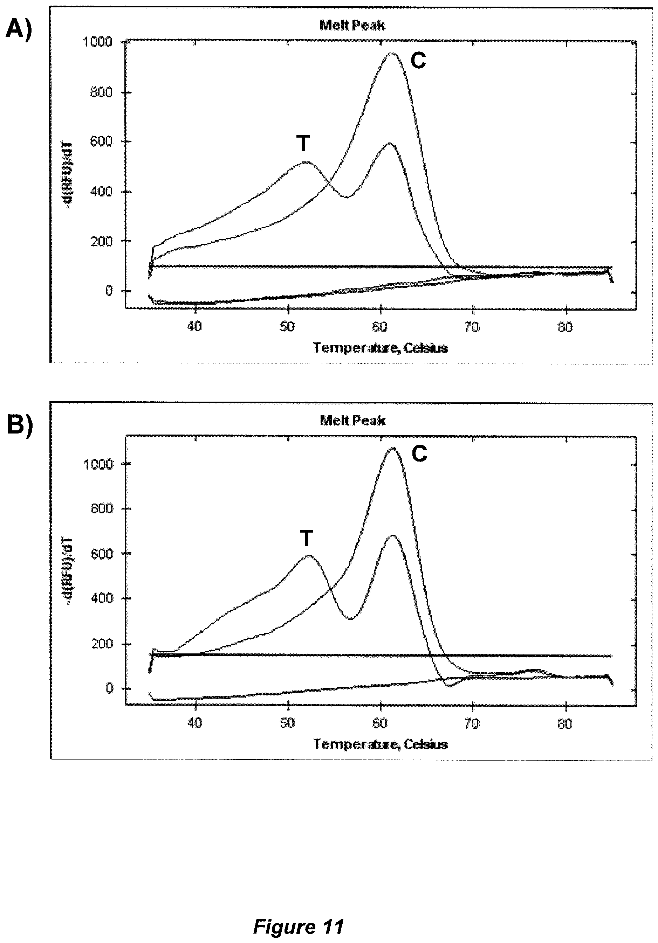

Extension of the CXCL12-PYR oligonucleotide and incorporation of the probe into amplified targets prevents detection of the mismatched T allele of the rs1746048 polymorphism (Figure 10) which is expected to appear at 52°C (see Figure 11). Any free probe available for target hybridisation preferentially binds to the fully complementary C allele. Decreasing the magnesium chloride concentration to 1.5mM reduces the magnitude of the extension peak allowing samples of C/C, C/T and T/T genotype to be clearly identified by melting curve analysis (data not shown).

The CXCL12-PYR oligonucleotide provides a second example of probe extension. In this instance 3'-5' exonuclease cleavage of the 3' Pyrene modification occurs in a singleplex PCR. Comparable results were generated with the Q5 Hot Start High-Fidelity DNA polymerase™ (data not shown).

The PrimerLisf™ tool (Kalendar *et al.* 2001) was used to investigate significant sequence complementarity between the oligonucleotides listed in Table 5 (to look for potential hybrids forming at amplification reaction temperatures). An oligonucleotide pairing involving the CXCL12-PYR oligonucleotide was not revealed, suggesting that removal of the PCR blocking modification by 3'-5' exonuclease activity and subsequent probe extension could occur independently of oligonucleotide dimer formation.

Experimental analysis of probe extension was performed by removing either the forward or reverse primer from the PCR to look for interactions with the probe oligonucleotide. Removing the CXCL12-F forward primer yielded 76.5°C melting peaks in the presence of template DNA or 73.5°C with No Template Controls.

The 76.5°C melting peak is generated when the 3' probe cap is removed and the CXCL12-PYR oligonucleotide acts as a primer to amplify a 51 bp product with the CXCL12-R reverse primer. Removing the CXCL12-R primer generated the 73.5°C melting peak in the presence of template DNA and with NTCs.

No melting peaks were generated when both forward and reverse primers were removed. Probe extension occurs in the absence of template nucleic acid and an obvious oligonucleotide interaction has not been found.

*Example 8*

A nucleotide sequence capable of forming a stem-loop structure was added to the 3' end of the CXCL12-PYR probe to prevent 3'-5' exonuclease removal of the 3' PCR blocking modification. The stem-loop structure was formed with the sequence 5'(ggccgAAAAcggcc)3', where lower and upper case nucleotides form the stem and loop, respectively. The CXCL12-3SL oligonucleotide has a 3' Pyrene dT Long modification to prevent PCR extension of the probe (Table 5). This probe was tested with the PCR formulation and thermal protocol described in example 7, replacing the CXCL12-PYR oligonucleotide.

The stem-loop sequence added to the 3' end of CXCL12-3SL oligonucleotide prevents removal of the Pyrene modification and subsequent extension of the probe. Extension peaks were not observed at 76.5°C with annealing and melting curve analysis, allowing clear detection of the T and C target alleles with peaks generated at 52°C and 61 °C, respectively (Figure 11).

*Example 9*

The ability of synthetic oligonucleotide sequences to form stem-loop structures was evaluated using the mfold™ program (Zuker, 2003). The $\Delta$G and $T_m$ values for secondary structures were also determined with mfold calculated using free energies based on nearest-neighbour thermodynamics (SantaLucia, 1998). The stability of hairpin structures depends on the length and sequence of the stem and loop components (Table 6).

**Table 6:** Sequence analysis using mfold to determine the ability to form stem-loop structures. $\Delta$G values were determined at 37°C and $T_m$ values calculated with 3mM magnesium chloride assuming a two-state model. Nucleotides that form the stem and loop components of the sequence are presented in lowercase and uppercase, respectively.

| Stem-loop sequence | $\Delta$G (KCal/mol) | $T_m$ (°C) |
|---|---|---|
| ggtgTTTTTTTcacc | -1.39 | 51.5 |
| ggtgTTTTTTcacc* | -1.69 | 54.8 |
| ggtgTTTTTcacc | -2.39 | 62.9 |
| ggtgTTTTcacc | -2.69 | 65.9 |
| ggtgTTTcacc | -1.29 | 53.9 |
| ggtGTTCacc | -0.02 | 37.2 |
| ggccgAAAAAAAcggcc | -4.87 | 77.2 |
| ggccgAAAAAAcggcc | -5.17 | 80.0 |
| ggccgAAAAAcggcc | -5.87 | 86.8 |
| ggccgAAAAcggcc* | -4.17 | 75.9 |
| ggccgAAAcggcc | -4.67 | 82.6 |
| ggccGAACggcc | -3.48 | 73.8 |
| tgcgTTTTTTTcgca | -2.50 | 59.2 |
| tgcgTTTTTTcgca | -2.80 | 62.1 |
| tgcgTTTTTcgca | -3.50 | 69.1 |
| tgcgTTTTcgca | -3.80 | 71.6 |
| tgcgTTTcgca | -2.40 | 62.8 |
| tgcGTTCgca | -0.41 | 41.8 |
| caggcAAATTAAgcctg | -3.59 | 70.4 |
| caggcAATTAAgcctg | -3.89 | 73.5 |
| caggcATTAAgcctg | -4.59 | 81.0 |
| caggcATTAgcctg | -4.39 | 78.8 |
| caggcATAgcctg | -3.39 | 71.2 |

(continued)

| Stem-loop sequence | ∆G (KCal/mol) | $T_m$ (°C) |
|---|---|---|
| caggCTAGcctg | -2.13 | 59.6 |

\* = used in the examples. Please note the 5'(TTTTTTTTTTCACC)3' sequence of P2-14nt (example 5) could not form a stable structure using mfold.

## REFERENCES

Ailenberg M, Silverman M. Controlled hot start and improved specificity in carrying out PCR utilizing touch-up and loop incorporated primers (TULIPS). Biotechniques 2000 29:1018-24

Ben Gaied N, Richardson JA, Singleton DG, Zhao Z, French D, Brown T. Endcapped HyBeacon probes for the analysis of human genetic polymorphisms related to warfarin metabolism. Org Biomol Chem. 2010 Jun 21;8(12):2728-34.

Breslauer KJ, Frank R, Blöcker H, Marky LA. Predicting DNA duplex stability from the base sequence. Proc Natl Acad Sci USA. 1986 Jun;83(11):3746-50.

El-Sagheer, A.H. and Brown, T. (2009) Synthesis and Polymerase Chain Reaction Amplification of DNA Strands Containing an Unnatural Triazole Linkage. J. Am. Chem. Soc., 131, 3958-3964.

El-Sagheer, A.H. and Brown, T. (2012) Click Nucleic Acid Ligation: Applications in Biology and Nanotechnology. Accounts Chem. Res., 45, 1258-1267.

El-Sagheer, A.H., Cheong, V.V. and Brown, T. (2011b) Rapid chemical ligation of oligonucleotides by the Diels-Alder reaction. Org. Biomol. Chem., 9, 232-235.

El-Sagheer, A.H., Sanzone, A.P., Gao, R., Tavassoli, A. and Brown, T. (2011a) Biocompatible artificial DNA linker that is read through by DNA polymerases and is functional in Escherichia coli. Proc. Natl. Acad. Sci. U. S. A., 108, 11338-11343.

French DJ, Archard CL, Andersen MT, McDowell DG. Ultra-rapid DNA analysis using HyBeacon probes and direct PCR amplification from saliva. Mol Cell Probes. 2002 Oct;16(5):319-26.

French DJ, Archard CL, Brown T, McDowell DG. HyBeacon probes: a new tool for DNA sequence detection and allele discrimination. Mol Cell Probes. 2001 Dec;15(6):363-74.

French DJ, Howard RL, Gale N, Brown T, McDowell DG, Debenham PG. Interrogation of short tandem repeats using fluorescent probes and melting curve analysis: a step towards rapid DNA identity screening. Forensic Sci Int Genet. 2008 Sep;2(4):333-9.

French DJ, Jones D, McDowell DG, Thomson JA, Debenham PG. Analysis of multiple single nucleotide polymorphisms closely positioned in the ovine PRNP gene using linear fluorescent probes and melting curve analysis. BMC Infect Dis. 2007 Aug 3;7:90.

Gerrard, S.R., Hardiman, C., Shelbourne, M., Nandhakumar, I., Norden, B. and Brown, T. (2012) A New Modular Approach to Nanoassembly: Stable and Addressable DNA Nanoconstructs via Orthogonal Click Chemistries. Acs Nano, 6, 9221-9228.

Kaboev OK, Luchkina LA, Tret'iakov AN, Bahrmand AR. Nucleic Acids Res. 2000 28:29:e94

Kalendar R, Lee D, Schulman AH. Java web tools for PCR, in silico PCR, and oligonucleotide assembly and analysis. Genomics. 2011 Aug;98(2):137-44.

Knemeyer JP, Marmé N, Sauer M. Probes for detection of specific DNA sequences at the single-molecule level. Anal Chem. 2000 Aug 15;72(16):3717-24.

Kumar, R., EI-Sagheer, A.H., Tumpane, J., Lincoln, P., Wilhelmsson, L.M. and Brown, T. (2007) Template-directed oligonucleotide strand ligation, covalent intramolecular DNA circularization and catenation using click chemistry. J. Am. Chem. Soc., 129, 6859-6864.

Nazarenko I, Lowe B, Darfler M, Ikonomi P, Schuster D, Rashtchian A. Multiplex quantitative PCR using self-quenched primers labeled with a single fluorophore. Nucleic Acids Res. 2002 May 1;30(9):e37.

Nazarenko IA, Bhatnagar SK, Hohman RJ. A closed tube format for amplification and detection of DNA based on energy transfer. Nucleic Acids Res. 1997 Jun 15;25(12):2516-21.

Notomi T, Okayama H, Masubuchi H, Yonekawa T, Watanabe K, Amino N, Hase T. Loop-mediated isothermal amplification of DNA. Nucleic Acids Res. 2000 Jun 15;28(12):E63.

Richardson JA, Gerowska M, Shelbourne M, French D, Brown T. Six-colour HyBeacon probes for multiplex genetic analysis. Chembiochem. 2010 Dec 10;11(18):2530-3.

SantaLucia J Jr. A unified view of polymer, dumbbell, and oligonucleotide DNA nearest-neighbor thermodynamics. Proc Natl Acad Sci USA. 1998 Feb 17;95(4):1460-5.

Satterfield BC, West JA, Caplan MR. Tentacle probes: eliminating false positives without sacrificing sensitivity. Nucleic Acids Res. 2007;35(10):e76.

Shelbourne, M., Brown, T. and EI-Sagheer, A.H. (2012) Fast and efficient DNA crosslinking and multiple orthogonal labelling by copper-free click chemistry. Chem. Commun., 48, 11184-11186.

Shelbourne, M., Chen, X., Brown, T. and EI-Sagheer, A.H. (2011) Fast copper-free click DNA ligation by the ring-strain promoted alkyne-azide cycloaddition reaction. Chem. Commun., 47, 6257-6259.

Sun L, Yu C and Irudayaraj J (2007) Surface-Enhanced Raman Scattering Based Nonfluorescent Probe for Multiplex DNA Detection. Anal Chem. 79 pp3981-3988

Thelwell N, Millington S, Solinas A, Booth J, Brown T. Mode of action and application of Scorpion primers to mutation detection. Nucleic Acids Res. 2000 Oct 1;28(19):3752-61.

Tyagi S, Bratu DP, Kramer FR. Multicolor molecular beacons for allele discrimination. Nat Biotechnol. 1998 Jan;16(1):49-53.

Tyagi S, Kramer FR. Molecular beacons: probes that fluoresce upon hybridization. Nat Biotechnol. 1996 Mar;14(3):303-8.

Wang X and Smirnov S (2009) Label-Free DNA Sensor Based on Surface Charge Modulated Ionic Conductance. ACS Nano. 3 pp1004-1010.

Whitcombe D, Theaker J, Guy SP, Brown T, Little S. Detection of PCR products using self-probing amplicons and fluorescence. Nat Biotechnol. 1999 Aug;17(8):804-7.

Zuker M. Mfold web server for nucleic acid folding and hybridization prediction. Nucleic Acids Res. 2003 Jul 1;31(13):3406-15.

## Claims

1. A single stranded oligonucleotide having a 5' end and a 3' end, said oligonucleotide comprising a first and second section, the first section being positioned 5' of the second section, wherein:

(i) the first section is labelled internally with at least two detectable labels, wherein the at least two detectable labels are the same, and is capable of hybridising to a target polynucleotide; and

(ii) the second section is not capable of hybridising to a target polynucleotide; said second section comprising a stem-loop structure comprising a first portion, a second portion and a third portion and wherein the second portion is located between the first and third portions, and the first and third portions are complementary to each other, wherein the stem-loop structure is not labelled with a detectable label;

and further wherein the oligonucleotide is (a) not cleaved by a 3'-5' exonuclease and (b) not extended by a polymerase, when used in a method of detecting a target polynucleotide.

2.  A method of detecting the presence of a target polynucleotide and/or sequence variations within the target polynucleotide in a sample of interest, comprising the steps of:

(i) providing a single stranded oligonucleotide having a 5' end and a 3' end, said oligonucleotide comprising a first and second section, the first section being positioned 5' of the second section; and wherein:

the first section is internally labelled with at least two detectable labels, wherein the at least two detectable labels are the same, and is capable of hybridising to a target polynucleotide; and

the second section is not capable of hybridising to a target polynucleotide; said second section comprising a stem-loop structure comprising a first portion, a second portion and a third portion and wherein the second portion is located between the first and third portions, and the first and third portions are complementary to each other, and wherein the stem-loop structure is not labelled with a detectable label;

(ii) exposing the sample of interest to the oligonucleotide of (i);

(iii) detecting a change in the detectable label, wherein a change in the detectable label indicates the presence of the target polynucleotide and/or the sequence variations within the target polynucleotide;

and wherein there is an absence of (a) cleavage of the oligonucleotide by a 3'-5' exonuclease and (b) extension of the oligonucleotide by a polymerase when the method is performed.

3.  The oligonucleotide of claim 1 or the method of claim 2 wherein the detectable labels are visually detectable labels, optionally wherein the detectable labels are present without an associated quencher.

4.  The oligonucleotide of claim 1 or 3 or the method of claim 2 or 3 wherein the second section is not capable of hybridising to the first section, optionally wherein the stem-loop structure does not disassociate when the oligonucleotide hybridises to the target polynucleotide, and/or wherein the first section is between 15 and 40 or between 18 and 30 nucleotide residues in length; and/or optionally wherein the second section is between 7 and 40 or between 12 and 22 nucleotide residues in length.

5.  The oligonucleotide of any of claims 1, 3 or 4 or the method of any of claims 2 to 4 wherein each of the first and third portions of the stem-loop structure comprise between 2 and 10 nucleotides or between 4 and 8 nucleotides, optionally wherein the second portion of the stem-loop structure comprises between 3 and 20 nucleotides or between 4 and 6 nucleotides.

6.  The oligonucleotide of any of claims 1 or 3 to 5 or the method of any of claims 2 to 5 wherein the oligonucleotide is modified at the 3' end with at least one modification to prevent extension by a polymerase and/or dimer formation.

7.  The oligonucleotide of any of claims 1 or 3 to 6 or the method of any of claims 2 to 6 wherein the stem-loop structure prevents (i) cleavage of the oligonucleotide by a 3'-5' exonuclease and/or (ii) extension of the oligonucleotide by a polymerase, optionally wherein the stem-loop structure and/or the at least one modification of the 3' end improve detection of nucleic acid target sequences in comparison to a control method using an oligonucleotide without the second section, and/or optionally wherein the first section and second section of the oligonucleotide are separated by a linker or spacer modification.

8.  The oligonucleotide of any of claims 1 or 3 to 7 or the method of any of claims 2 to 7 wherein the melting temperature ($T_m$) of the second oligonucleotide section is between 55°C and 95°C, optionally wherein the stability of the second oligonucleotide section is between -1 kcal/mol and -10 kcal/mol or between -2 kcal/mol and -6 kcal/mol..

9. The oligonucleotide of any of claims 1 or 3 to 8 or the method of any of claims 2 to 8 wherein the detectable label is a fluorophore or a dye, optionally wherein the detectable label is fluorescein dT, SIMA dT, TAMRA dT, Texas Red or ATTO 647N, and/or optionally wherein the first section is labelled with three or more; or four or more detectable labels.

10. The method of any of claims 2 to 9 wherein step (iii) is undertaken using either melting curve analysis or annealing curve analysis, optionally wherein step (iii) is used in target detection, SNP genotyping, or detection of length polymorphisms and repetitive sequences, and/or optionally wherein the method is for use in conjunction with an isothermal nucleic acid amplification methodology such as Loop-mediated isothermal amplification (LAMP) method.

11. Use of an oligonucleotide as defined in any of claims 1 and 3 to 9 in a method for detecting the presence of a target polynucleotide and/or sequence variations within the target polynucleotide in a sample of interest, optionally wherein the use is for target detection, SNP genotyping, or detection of length polymorphisms and repetitive sequences.

12. A method of making an oligonucleotide as defined in any of claims 1 and 3 to 9, comprising the steps of:

(a) providing a first oligonucleotide labelled with at least two detectable labels and is capable of hybridising to a target polynucleotide;
(b) providing a second oligonucleotide which is not capable of hybridising to a target polynucleotide; said second oligonucleotide being capable of forming a stem-loop structure comprising a first portion, a second portion and a third portion and wherein the second portion is located between the first and third portions, and the first and third portions are complementary to each other;
(c) ligating the first and second oligonucleotides to form a single oligonucleotide, with the first oligonucleotide being positioned 5' of the second oligonucleotide.

13. An oligonucleotide library comprising a plurality of oligonucleotides as defined in any of claims 1 and 3 to 9, optionally wherein the plurality of oligonucleotides may each comprise a different detectable label, and/or optionally wherein the plurality of oligonucleotides are attached to a solid support.

14. A kit of parts comprising:

(a) an oligonucleotide as defined in any of claims 1 and 3 to 11; and
(b) instructions for use;

optionally wherein the kit further comprises one or more selected from reaction buffer (for PCR or isothermal amplification), dNTPs, oligonucleotide primers, enzyme and further additives including but not limited to $MgCl_2$, Bovine Serum Albumin (BSA), Dimethyl Sulfoxide (DMSO), Betaine, Tween-20 and carrier RNA.

15. A kit of parts comprising:

(a) the first section of an oligonucleotide as defined in any of claims 1 and 3 to 9;
(b) the second section of an oligonucleotide as defined in any of claims 1 and 3 to 9; and
(c) instructions for use;

optionally wherein the kit further comprises means for conjugating the first and second sections to form a single oligonucleotide, and/or optionally wherein the kit further comprises one or more selected from reaction buffer (for PCR or isothermal amplification), dNTPs, oligonucleotide primers, enzyme and further additives including but not limited to $MgCl_2$, Bovine Serum Albumin (BSA), Dimethyl Sulfoxide (DMSO), Betaine, Tween-20 and carrier RNA.

**Patentansprüche**

1. Einzelsträngiges Oligonukleotid mit einem 5'-Ende und einem 3'-Ende, wobei das Oligonukleotid einen ersten und zweiten Abschnitt umfasst, wobei der erste Abschnitt an 5' des zweiten Abschnitts angeordnet ist, wobei:

(i) der erste Abschnitt intern mit wenigstens zwei nachweisbaren Markierungen markiert ist, wobei die wenigstens zwei nachweisbaren Markierungen gleich sind, und zum Hybridisieren an ein Ziel-Polynukleotid in der Lage ist; und

(ii) der zweite Abschnitt nicht zum Hybridisieren an ein Ziel-Polynukleotid in der Lage ist; wobei der zweite Abschnitt eine Stamm-Schleife-Struktur, umfassend einen ersten Teil, einen zweiten Teil und einen dritten Teil und wobei der zweite Teil sich zwischen dem ersten und dritten Teil befindet und der erste und dritte Teil komplementär zueinander sind, umfasst, wobei die Stamm-Schleife-Struktur nicht mit einer nachweisbaren Markierung markiert ist;

und wobei ferner das Oligonukleotid (a) nicht durch eine 3'-5'-Exonuklease gespalten wird und (b) nicht durch eine Polymerase verlängert wird, wenn es in einem Verfahren zum Nachweisen eines Ziel-Polynukleotids verwendet wird.

**2.** Verfahren zum Nachweisen des Vorhandenseins eines Ziel-Polynukleotids und/oder von Sequenzvariationen innerhalb des Ziel-Polynukleotids in einer interessierenden Probe, das die folgenden Schritte umfasst:

(i) Bereitstellen eines einzelsträngigen Oligonukleotids mit einem 5'-Ende und einem 3'-Ende, wobei das Oligonukleotid einen ersten und zweiten Abschnitt umfasst, wobei der erste Abschnitt an 5' des zweiten Abschnitts angeordnet ist; und wobei:

der erste Abschnitt intern mit wenigstens zwei nachweisbaren Markierungen markiert ist, wobei die wenigstens zwei nachweisbaren Markierungen gleich sind, und zum Hybridisieren an ein Ziel-Polynukleotid in der Lage ist; und der zweite Abschnitt nicht zum Hybridisieren an ein Ziel-Polynukleotid in der Lage ist; wobei der zweite Abschnitt eine Stamm-Schleife-Struktur, umfassend einen ersten Teil, einen zweiten Teil und einen dritten Teil und wobei der zweite Teil sich zwischen dem ersten und dritten Teil befindet und der erste und dritte Teil komplementär zueinander sind, umfasst, und wobei die Stamm-Schleife-Struktur nicht mit einer nachweisbaren Markierung markiert ist;

(ii) Aussetzen der interessierenden Probe dem Oligonukleotid von (i);
(iii) Nachweisen einer Änderung in der nachweisbaren Markierung, wobei eine Änderung in der nachweisbaren Markierung das Vorhandensein des Ziel-Polynukleotids und/oder der Sequenzvariationen innerhalb des Ziel-Polynukleotids anzeigt;

und wobei eine Abwesenheit (a) einer Spaltung des Oligonukleotids durch eine 3'-5'-Exonuklease und (b) einer Verlängerung des Oligonukleotids durch eine Polymerase vorliegt, wenn das Verfahren durchgeführt wird.

**3.** Oligonukleotid nach Anspruch 1 oder Verfahren nach Anspruch 2, wobei die nachweisbaren Markierungen optisch nachweisbare Markierungen sind, wobei die nachweisbaren Markierungen optional ohne einen zugeordneten Quencher vorhanden sind.

**4.** Oligonukleotid nach Anspruch 1 oder 3 oder Verfahren nach Anspruch 2 oder 3, wobei der zweite Abschnitt nicht zum Hybridisieren an den ersten Abschnitt in der Lage ist, wobei die Stamm-Schleife-Struktur optional nicht dissoziiert, wenn das Oligonukleotid an das Ziel-Polynukleotid hybridisiert, und/oder wobei der erste Abschnitt zwischen 15 und 40 oder zwischen 18 und 30 Nukleotidreste lang ist; und/oder wobei der zweite Abschnitt optional zwischen 7 und 40 oder zwischen 12 und 22 Nukleotidreste lang ist.

**5.** Oligonukleotid nach einem der Ansprüche 1, 3 oder 4 oder Verfahren nach einem der Ansprüche 2 bis 4, wobei der erste und dritte Teil der Stamm-Schleife-Struktur jeweils zwischen 2 und 10 Nukleotide oder zwischen 4 und 8 Nukleotide umfassen, wobei der zweite Teil der Stamm-Schleife-Struktur optional zwischen 3 und 20 Nukleotide oder zwischen 4 und 6 Nukleotide umfasst.

**6.** Oligonukleotid nach einem der Ansprüche 1 oder 3 bis 5 oder Verfahren nach einem der Ansprüche 2 bis 5, wobei das Oligonukleotid am 3'-Ende mit wenigstens einer Modifikation modifiziert ist, um eine Verlängerung durch eine Polymerase und/oder eine Dimerbildung zu verhindern.

**7.** Oligonukleotid nach einem der Ansprüche 1 oder 3 bis 6 oder Verfahren nach einem der Ansprüche 2 bis 6, wobei die Stamm-Schleife-Struktur (i) eine Spaltung des Oligonukleotids durch eine 3'-5'-Exonuklease und/oder (ii) eine Verlängerung des Oligonukleotids durch eine Polymerase verhindert, wobei die Stamm-Schleife-Struktur und/oder die wenigstens eine Modifikation des 3'-Endes optional einen Nachweis von Nukleinsäure-Zielsequenzen im Vergleich zu einem Kontrollverfahren, das ein Oligonukleotid ohne den zweiten Abschnitt verwendet, verbessert und/oder wobei der erste Abschnitt und zweite Abschnitt des Oligonukleotids optional durch eine Linker- oder Spacer-Modifikation getrennt sind.

**EP 3 039 160 B1**

8. Oligonukleotid nach einem der Ansprüche 1 oder 3 bis 7 oder Verfahren nach einem der Ansprüche 2 bis 7, wobei die Schmelztemperatur ($T_m$) des zweiten Oligonukleotidabschnitts zwischen 55 °C und 95 °C liegt, wobei die Stabilität des zweiten Oligonukleotidabschnitts optional zwischen -1 kcal/mol und -10 kcal/mol oder zwischen -2 kcal/mol und -6 kcal/mol liegt.

9. Oligonukleotid nach einem der Ansprüche 1 oder 3 bis 8 oder Verfahren nach einem der Ansprüche 2 bis 8, wobei die nachweisbare Markierung ein Fluorophor oder ein Farbstoff ist, wobei die nachweisbare Markierung optional Fluorescein dT, SIMA dT, TAMRA dT, Texas Red oder ATTO 647N ist, und/oder wobei der erste Abschnitt optional mit drei oder mehr; oder vier oder mehr nachweisbaren Markierungen markiert ist.

10. Verfahren nach einem der Ansprüche 2 bis 9, wobei Schritt (iii) unter Verwendung einer Schmelzkurvenanalyse oder einer Glühkurvenanalyse durchgeführt wird, wobei Schritt (iii) optional bei einem Ziel-Nachweis, einer SNP-Genotypisierung oder einem Nachweis von Längenpolymorphismen und sich wiederholenden Sequenzen verwendet wird und/oder wobei das Verfahren optional zur Verwendung im Zusammenhang mit einer Vorgehensweise zur isothermen Nukleinsäureamplifikation, wie etwa einem Verfahren der schleifenvermittelten isothermen Amplifikation (LAMP - *loop-mediated isothermal amplification*), dient.

11. Verwendung eines Oligonukleotids nach einem der Ansprüche 1 und 3 bis 9 in einem Verfahren zum Nachweisen des Vorhandenseins eines Ziel-Polynukleotids und/oder von Sequenzvariationen innerhalb des Ziel-Polynukleotids in einer interessierenden Probe, wobei die Verwendung optional zum Ziel-Nachweis, zur SNP-Genotypisierung oder zum Nachweis von Längenpolymorphismen und sich wiederholenden Sequenzen dient.

12. Verfahren zum Herstellen eines Oligonukleotids nach einem der Ansprüche 1 und 3 bis 9, das die folgenden Schritte umfasst:

    (a) Bereitstellen eines ersten Oligonukleotids, das mit wenigstens zwei nachweisbaren Markierungen markiert ist und zum Hybridisieren an ein Ziel-Polynukleotid in der Lage ist;
    (b) Bereitstellen eines zweiten Oligonukleotids, das nicht zum Hybridisieren an ein Ziel-Polynukleotid in der Lage ist; wobei das zweite Oligonukleotid zum Ausbilden einer Stamm-Schleife-Struktur, umfassend einen ersten Teil, einen zweiten Teil und einen dritten Teil und wobei der zweite Teil sich zwischen dem ersten und dritten Teil befindet und der erste und dritte Teil komplementär zueinander sind, in der Lage ist;
    (c) Verknüpfen des ersten und zweiten Oligonukleotids, um ein einziges Oligonukleotid auszubilden, wobei das erste Oligonukleotid an 5' des zweiten Oligonukleotids angeordnet ist.

13. Oligonukleotid-Bibliothek, die mehrere Oligonukleotide nach einem der Ansprüche 1 und 3 bis 9 umfasst, wobei die mehreren Oligonukleotide optional jeweils eine andere nachweisbare Markierung umfassen können und/oder wobei die mehreren Oligonukleotide optional an einen festen Träger gebunden sind.

14. Set von Teilen, Folgendes umfassend:

    (a) ein Oligonukleotid nach einem der Ansprüche 1 und 3 bis 11; und
    (b) eine Benutzungsanleitung;

    wobei das Set optional ferner eines oder mehrere, ausgewählt aus einem Reaktionspuffer (für PCR oder isotherme Amplifikation), dNTPs, Oligonukleotid-Primern, Enzym und weiteren Zusatzstoffen, darunter, jedoch nicht beschränkt auf, $MgCl_2$, Rinderserumalbumin (BSA - *Bovine Serum Albumin*), Dimethylsulfoxid (DMSO), Betain, Tween-20 und Träger-RNA, umfasst.

15. Set von Teilen, Folgendes umfassend:

    (a) den ersten Abschnitt eines Oligonukleotids nach einem der Ansprüche 1 und 3 bis 9;
    (b) den zweiten Abschnitt eines Oligonukleotids nach einem der Ansprüche 1 und 3 bis 9; und
    (c) eine Benutzungsanleitung;

    wobei das Set optional ferner Mittel zum Konjugieren des ersten und zweiten Abschnitts zum Ausbilden eines einzigen Oligonukleotids umfasst und/oder wobei das Set optional ferner eines oder mehrere, ausgewählt aus einem Reaktionspuffer (für PCR oder isotherme Amplifikation), dNTPs, Oligonukleotid-Primern, Enzym und weiteren Zusatzstoffen, darunter, jedoch nicht beschränkt auf, $MgCl_2$, Rinderserumalbumin (BSA - *Bovine Serum Albumin*),

23

Dimethylsulfoxid (DMSO), Betain, Tween-20 und Träger-RNA, umfasst.

**Revendications**

1. Oligonucléotide simple brin ayant une extrémité 5' et une extrémité 3', ledit oligonucléotide comprenant une première section et une deuxième section, la première section étant en position 5' de la deuxième section, dans lequel :

   (i) la première section est marquée au niveau interne avec au moins deux marqueurs détectables, dans laquelle les au moins deux marqueurs détectables sont identiques, et est capable de s'hybrider à un polynucléotide cible ; et
   (ii) la deuxième section n'est pas capable de s'hybrider à un polynucléotide cible ; ladite deuxième section comprenant une structure tige-boucle comprenant une première partie, une deuxième partie et une troisième partie et dans laquelle la deuxième partie est située entre les première et troisième parties, et les première et troisième parties sont complémentaires l'une de l'autre, dans laquelle la structure tige-boucle n'est pas marquée avec un marqueur détectable ;

   et en outre dans lequel l'oligonucléotide n'est (a) pas clivé par une exonucléase 3'-5' et (b) pas allongé par une polymérase, lorsqu'il est utilisé dans un procédé de détection d'un polynucléotide cible.

2. Procédé de détection de la présence d'un polynucléotide cible et/ou de variations de séquence au sein du polynucléotide cible dans un échantillon d'intérêt, comprenant les étapes consistant à :

   (i) fournir un oligonucléotide simple brin ayant une extrémité 5' et une extrémité 3', ledit oligonucléotide comprenant une première section et une deuxième section, la première section étant en position 5' de la deuxième section, et dans lequel :

   la première section est marquée au niveau interne avec au moins deux marqueurs détectables, dans laquelle les au moins deux marqueurs détectables sont identiques, et est capable de s'hybrider à un polynucléotide cible ; et
   la deuxième section n'est pas capable de s'hybrider à un polynucléotide cible ; ladite deuxième section comprenant une structure tige-boucle comprenant une première partie, une deuxième partie et une troisième partie et dans laquelle la deuxième partie est située entre les première et troisième parties, et les première et troisième parties sont complémentaires l'une de l'autre, et dans laquelle la structure tige-boucle n'est pas marquée avec un marqueur détectable ;

   (ii) exposer l'échantillon d'intérêt à l'oligonucléotide de (i) ;
   (iii) détecter une modification dans le marqueur détectable, où une modification dans le marqueur détectable indique la présence du polynucléotide cible et/ou des variations de séquence au sein du polynucléotide cible ;

   et dans lequel il y a une absence (a) de clivage de l'oligonucléotide par une exonucléase 3'-5' et (b) d'allongement de l'oligonucléotide par une polymérase lorsque le procédé est effectué.

3. Oligonucléotide selon la revendication 1 ou procédé selon la revendication 2, dans lequel les marqueurs détectables sont des marqueurs visuellement détectables, facultativement dans lequel les marqueurs détectables sont présents sans désactiveur associé.

4. Oligonucléotide selon la revendication 1 ou 3 ou procédé selon la revendication 2 ou 3, dans lequel la deuxième section n'est pas capable de s'hybrider à la première section, facultativement dans lequel la structure tige-boucle ne se dissocie pas lorsque l'oligonucléotide s'hybride au polynucléotide cible, et/ou dans lequel la première section a une longueur comprise entre 15 et 40 ou entre 18 et 30 résidus nucléotidiques ; et/ou facultativement dans lequel la deuxième section a une longueur comprise entre 7 et 40 ou entre 12 et 22 résidus nucléotidiques.

5. Oligonucléotide selon l'une quelconque des revendications 1, 3 ou 4 ou procédé selon l'une quelconque des revendications 2 à 4, dans lequel chacune des première et troisième parties de la structure tige-boucle comprend entre 2 et 10 nucléotides ou entre 4 et 8 nucléotides, facultativement dans lequel la deuxième partie de la structure tige-boucle comprend entre 3 et 20 nucléotides ou entre 4 et 6 nucléotides.

**6.** Oligonucléotide selon l'une quelconque des revendications 1 ou 3 à 5 ou procédé selon l'une quelconque des revendications 2 à 5, dans lequel l'oligonucléotide est modifié au niveau de l'extrémité 3' avec au moins une modification pour empêcher l'allongement par une polymérase et/ou la formation de dimère.

**7.** Oligonucléotide selon l'une quelconque des revendications 1 ou 3 à 6 ou procédé selon l'une quelconque des revendications 2 à 6, dans lequel la structure tige-boucle empêche (i) le clivage de l'oligonucléotide par une exonucléase 3'-5' et/ou (ii) l'allongement de l'oligonucléotide par une polymérase, facultativement dans lequel la structure tige-boucle et/ou l'au moins une modification de l'extrémité 3' améliore la détection des séquences cibles d'acide nucléique par comparaison avec un procédé de contrôle utilisant un oligonucléotide sans deuxième section, et/ou facultativement dans lequel la première section et la deuxième section de l'oligonucléotide sont séparées par une modification de type lieur ou espaceur.

**8.** Oligonucléotide selon l'une quelconque des revendications 1 ou 3 à 7 ou procédé selon l'une quelconque des revendications 2 à 7, dans lequel la température de fusion ($T_m$) de la deuxième section oligonucléotidique est comprise entre 55 °C et 95 °C, facultativement dans lequel la stabilité de la deuxième section oligonucléotidique est comprise entre -1 kcal/mole et -10 kcal/mole ou entre - 2 kcal/mole et -6 kcal/mole.

**9.** Oligonucléotide selon l'une quelconque des revendications 1 ou 3 à 8 ou procédé selon l'une quelconque des revendications 2 à 8, dans lequel le marqueur détectable est un fluorophore ou un colorant, facultativement dans lequel le marqueur détectable est la fluorescéine dT, SIMA dT, TAMRA dT, Texas Red ou ATTO 647N, et/ou facultativement dans lequel la première section est marquée avec trois marqueurs détectables ou plus ; ou quatre marqueurs détectables ou plus.

**10.** Procédé selon l'une quelconque des revendications 2 à 9, dans lequel l'étape (iii) est réalisée en utilisant l'analyse de la courbe de fusion ou l'analyse de la courbe d'annelage, facultativement dans lequel l'étape (iii) est utilisée dans la détection de cibles, le génotypage SNP ou la détection de polymorphismes de longueur et de séquences répétitives, et/ou facultativement dans lequel le procédé est pour une utilisation en combinaison avec une technique d'amplification isotherme des acides nucléiques telle que le procédé d'amplification isotherme médiée par des boucles (LAMP).

**11.** Utilisation d'un oligonucléotide tel que défini dans l'une quelconque des revendications 1 et 3 à 9 dans un procédé de détection
de la présence d'un polynucléotide cible et/ou de variations de séquence au sein du polynucléotide cible dans un échantillon d'intérêt, facultativement laquelle utilisation est pour la détection de cibles, le génotypage SNP ou la détection de polymorphismes de longueur et de séquences répétitives.

**12.** Procédé de production d'un oligonucléotide tel que défini dans l'une quelconque des revendications 1 et 3 à 9, comprenant les étapes consistant à :

    (a) fournir un premier oligonucléotide marqué avec au moins deux marqueurs détectables et est capable de s'hybrider à un polynucléotide cible ;
    (b) fournir un deuxième oligonucléotide qui n'est pas capable de s'hybrider à un polynucléotide cible ; ledit deuxième oligonucléotide étant capable de former une structure tige-boucle comprenant une première partie, une deuxième partie et une troisième partie et dans lequel la deuxième partie est située entre les première et troisième parties, et les première et troisième parties sont complémentaires l'une de l'autre ;
    (c) ligaturer les premier et deuxième oligonucléotides pour former un oligonucléotide simple brin, avec le premier oligonucléotide en position 5' du deuxième oligonucléotide.

**13.** Banque d'oligonucléotides comprenant une pluralité d'oligonucléotides tels que définis dans l'une quelconque des revendications 1 et 3 à 9, facultativement dans laquelle la pluralité d'oligonucléotides peut chacune comprendre un marqueur détectable différent, et/ou facultativement dans laquelle la pluralité d'oligonucléotides est fixée à un support solide.

**14.** Ensemble d'éléments comprenant :

    (a) un oligonucléotide tel que défini dans l'une quelconque des revendications 1 et 3 à 11 ; et
    (b) des instructions d'utilisation ;

facultativement lequel ensemble comprend en outre un ou plusieurs produits choisis parmi le tampon de réaction (pour la PCR ou l'amplification isotherme), les dNTP, les amorces oligonucléotidiques, les enzymes et d'autres additifs tels que, entre autres, le $MgCl_2$, la sérumalbumine bovine (SAB), le diméthylsulfoxyde (DMSO), la bétaïne, le Tween-20 et l'ARN vecteur.

15. Ensemble d'éléments comprenant :

(a) la première section d'un oligonucléotide tel que défini dans l'une quelconque des revendications 1 et 3 à 9 ;
(b) la deuxième section d'un oligonucléotide tel que défini dans l'une quelconque des revendications 1 et 3 à 9 ; et
(c) des instructions d'utilisation ;

facultativement lequel ensemble comprend en outre un ou plusieurs moyens de conjugaison des première et deuxième sections pour former un oligonucléotide simple brin et/ou facultativement lequel ensemble comprend un ou plusieurs produits choisis parmi le tampon de réaction (pour la PCR ou l'amplification isotherme), les dNTP, les amorces oligonucléotidiques, les enzymes et d'autres additifs tels que, entre autres, le $MgCl_2$, la sérumalbumine bovine (SAB), le diméthylsulfoxyde (DMSO), la bétaïne, le Tween-20 et l'ARN vecteur.

**Figure 1**

*Figure 2*

*Figure 3*

*Figure 4*

*Figure 5*

*Figure 6*

*Figure 7*

Figure 8

*Figure 9*

*Figure 10*

Figure 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0173118 A
- WO 07010268 A
- US 20090305264 A
- WO 2010101947 A
- US 20070015176 A
- US 20060216692 A
- US 20060088856 A
- WO 2006020933 A
- WO 2005007818 A
- WO 9924621 A
- EP 0881302 A
- WO 9802449 A
- WO 0142505 A
- WO 0186001 A
- WO 2007010268 A
- GB 2012050645 W

### Non-patent literature cited in the description

- *Biotechniques,* 2000, vol. 29, 1018-24
- **BEN GAIED N ; RICHARDSON JA ; SINGLETON DG ; ZHAO Z ; FRENCH D ; BROWN T.** Endcapped HyBeacon probes for the analysis of human genetic polymorphisms related to warfarin metabolism. *Org Biomol Chem.,* 21 June 2010, vol. 8 (12), 2728-34
- **BRESLAUER KJ ; FRANK R ; BLÖCKER H ; MARKY LA.** Predicting DNA duplex stability from the base sequence. *Proc Natl Acad Sci USA.,* 1986, vol. 83 (11), 3746-50
- **EI-SAGHEER, A.H. ; BROWN, T.** Synthesis and Polymerase Chain Reaction Amplification of DNA Strands Containing an Unnatural Triazole Linkage. *J. Am. Chem. Soc.,* 2009, vol. 131, 3958-3964
- **EI-SAGHEER, A.H. ; BROWN, T.** Click Nucleic Acid Ligation: Applications in Biology and Nanotechnology. *Accounts Chem. Res.,* 2012, vol. 45, 1258-1267
- **EI-SAGHEER, A.H. ; CHEONG, V.V. ; BROWN, T.** Rapid chemical ligation of oligonucleotides by the Diels-Alder reaction. *Org. Biomol. Chem.,* 2011, vol. 9, 232-235
- **EI-SAGHEER, A.H. ; SANZONE, A.P. ; GAO, R. ; TAVASSOLI, A. ; BROWN, T.** Biocompatible artificial DNA linker that is read through by DNA polymerases and is functional in Escherichia coli. *Proc. Natl. Acad. Sci. U. S. A.,* 2011, vol. 108, 11338-11343
- **FRENCH DJ ; ARCHARD CL ; ANDERSEN MT ; MCDOWELL DG.** Ultra-rapid DNA analysis using Hy-Beacon probes and direct PCR amplification from saliva. *Mol Cell Probes,* October 2002, vol. 16 (5), 319-26
- **FRENCH DJ ; ARCHARD CL ; BROWN T ; MC-DOWELL DG.** HyBeacon probes: a new tool for DNA sequence detection and allele discrimination. *Mol Cell Probes,* December 2001, vol. 15 (6), 363-74
- **FRENCH DJ ; HOWARD RL ; GALE N ; BROWN T ; MCDOWELL DG ; DEBENHAM PG.** Interrogation of short tandem repeats using fluorescent probes and melting curve analysis: a step towards rapid DNA identity screening. *Forensic Sci Int Genet,* September 2008, vol. 2 (4), 333-9
- **FRENCH DJ ; JONES D ; MCDOWELL DG ; THOMSON JA ; DEBENHAM PG.** Analysis of multiple single nucleotide polymorphisms closely positioned in the ovine PRNP gene using linear fluorescent probes and melting curve analysis. *BMC Infect Dis.,* 03 August 2007, vol. 7, 90
- **GERRARD, S.R. ; HARDIMAN, C. ; SHELBOURNE, M. ; NANDHAKUMAR, I. ; NORDEN, B. ; BROWN, T.** A New Modular Approach to Nanoassembly: Stable and Addressable DNA Nanoconstructs via Orthogonal Click Chemistries. *Acs Nano,* 2012, vol. 6, 9221-9228
- **KABOEV OK ; LUCHKINA LA ; TRET'IAKOV AN ; BAHRMAND AR.** *Nucleic Acids Res.,* 2000, vol. 28 (29), e94
- **KALENDAR R ; LEE D ; SCHULMAN AH.** Java web tools for PCR, in silico PCR, and oligonucleotide assembly and analysis. *Genomics,* August 2011, vol. 98 (2), 137-44
- **KNEMEYER JP ; MARMÉ N ; SAUER M.** Probes for detection of specific DNA sequences at the single-molecule level. *Anal Chem.,* 15 August 2000, vol. 72 (16), 3717-24

- **KUMAR, R. ; EI-SAGHEER, A.H. ; TUMPANE, J. ; LINCOLN, P. ; WILHELMSSON, L.M. ; BROWN, T.** Template-directed oligonucleotide strand ligation, covalent intramolecular DNA circularization and catenation using click chemistry. *J. Am. Chem. Soc.,* 2007, vol. 129, 6859-6864
- **NAZARENKO I ; LOWE B ; DARFLER M ; IKONOMI P ; SCHUSTER D ; RASHTCHIAN A.** Multiplex quantitative PCR using self-quenched primers labeled with a single fluorophore. *Nucleic Acids Res.,* 01 May 2002, vol. 30 (9), e37
- **NAZARENKO IA ; BHATNAGAR SK ; HOHMAN RJ.** A closed tube format for amplification and detection of DNA based on energy transfer. *Nucleic Acids Res.,* 15 June 1997, vol. 25 (12), 2516-21
- **NOTOMI T ; OKAYAMA H ; MASUBUCHI H ; YONEKAWA T ; WATANABE K ; AMINO N ; HASE T.** Loop-mediated isothermal amplification of DNA. *Nucleic Acids Res.,* 15 June 2000, vol. 28 (12), E63
- **RICHARDSON JA ; GEROWSKA M ; SHELBOURNE M ; FRENCH D ; BROWN T.** Six-colour HyBeacon probes for multiplex genetic analysis. *Chembiochem,* 10 December 2010, vol. 11 (18), 2530-3
- **SANTALUCIA J JR.** A unified view of polymer, dumbbell, and oligonucleotide DNA nearest-neighbor thermodynamics. *Proc Natl Acad Sci USA.,* 17 February 1998, vol. 95 (4), 1460-5
- **SATTERFIELD BC ; WEST JA ; CAPLAN MR.** Tentacle probes: eliminating false positives without sacrificing sensitivity. *Nucleic Acids Res.,* 2007, vol. 35 (10), e76
- **SHELBOURNE, M. ; BROWN, T. ; EI-SAGHEER, A.H.** Fast and efficient DNA crosslinking and multiple orthogonal labelling by copper-free click chemistry. *Chem. Commun.,* 2012, vol. 48, 11184-11186
- **SHELBOURNE, M. ; CHEN, X. ; BROWN, T. ; EI-SAGHEER, A.H.** Fast copper-free click DNA ligation by the ring-strain promoted alkyne-azide cycloaddition reaction. *Chem. Commun.,* 2011, vol. 47, 6257-6259
- **SUN L ; YU C ; IRUDAYARAJ J.** Surface-Enhanced Raman Scattering Based Nonfluorescent Probe for Multiplex DNA Detection. *Anal Chem.,* 2007, vol. 79, 3981-3988
- **THELWELL N ; MILLINGTON S ; SOLINAS A ; BOOTH J ; BROWN T.** Mode of action and application of Scorpion primers to mutation detection. *Nucleic Acids Res.,* 01 October 2000, vol. 28 (19), 3752-61
- **TYAGI S ; BRATU DP ; KRAMER FR.** Multicolor molecular beacons for allele discrimination. *Nat Biotechnol.,* January 1998, vol. 16 (1), 49-53
- **TYAGI S ; KRAMER FR.** Molecular beacons: probes that fluoresce upon hybridization. *Nat Biotechnol.,* March 1996, vol. 14 (3), 303-8
- **WANG X ; SMIRNOV S.** Label-Free DNA Sensor Based on Surface Charge Modulated Ionic Conductance. *ACS Nano,* 2009, vol. 3, 1004-1010
- **WHITCOMBE D ; THEAKER J ; GUY SP ; BROWN T ; LITTLE S.** Detection of PCR products using self-probing amplicons and fluorescence. *Nat Biotechnol,* August 1999, vol. 17 (8), 804-7
- **ZUKER M.** Mfold web server for nucleic acid folding and hybridization prediction. *Nucleic Acids Res.,* 01 July 2003, vol. 31 (13), 3406-15